# EUROPEAN PATENT APPLICATION

(11) **EP 2 481 811 A1**
(43) Date of publication of application: **01.08.2012**
(21) Application number: 11152285.0
(22) Date of filing: 26.01.2011
(51) Int. Cl.: C12Q 1/68

(54) **Analysis of the methylation pattern of the alpha-synuclein gene from DNA of peripheral blood monocytes for diagnosing Parkinson's disease**

(71) Applicant: Rheinische Friedrich-Wilhelms Universität, 53113 Bonn (DE)
(72) Inventor: Wüllner, Ullrich, 53343 Wachtberg (DE); Schmitt, Ina, 58239 Schwerte (DE)
(74) Representative: Michalski Hüttermann & Partner

(57) **Abstract**

The invention pertains to the use of DNA from peripheral blood monocytes for determining the methylation pattern of the SNCA gene in diagnosing and/or assessing a patient's risk of developing Parkinson's disease, and further provides a method of determining the methylation pattern of the human SNCA gene for diagnosing and/or assessing a patient's risk of developing Parkinson's disease, wherein the SNCA gene is obtained from DNA of peripheral blood monocytes of said patient.

SEQUENCE LISTING

<110>  Rheinische Friedrich-Wilhelms-Universität

<120>  Analysis of the methylation pattern of the alpha-synuclein gene
       from DNA of peripheral blood monocytes for diagnosing Parkinson's disease

<130>  UD 40267

<160>  898

<170>  PatentIn version 3.5

<210>  1
<211>  444
<212>  DNA
<213>  Homo sapiens

<400>  1
ggagcctaag gaaagagact tgacctggct ttcgtcctgc ttctgatatt cccttctcca      60

caagggctga gagattaggc tgcttctccg ggatccgctt ttccccggga aacgcgagga     120

tgctccatgg agcgtgagca tccaactttt ctctcacata aaatctgtct gcccgctctc     180

ttggtttttc tctgtaaagt aagcaagctg cgtttggcaa ataatgaaat ggaagtgcaa     240

ggaggccaag tcaacaggtg gtaacgggtt aacaagtgct ggcgcggggt ccgctagggt     300

ggaggctgag aacgccccct cgggtggctg gcgcggggtt ggagacggcc cgcgagtgtg     360

agcggcgcct gctcagggta gatagctgag ggcgggggtg gatgttggat ggattagaac     420

catcacactt gggcctgctg tttg                                            444


<210>  2
<211>  25
<212>  DNA
<213>  Artificial Sequence

<220>
<223>  Forward primer for PCR amplification of bisulfite treated SNCA
       (-926/-483) DNA

<400>  2
ggagtttaag gaaagagatt tgatt                                            25


<210>  3
<211>  25
<212>  DNA
<213>  Artificial Sequence

<220>
<223>  reverse Primer for PCR amplification of SNCA(-926/-483) DNA

<400>  3
caaacaacaa acccaaatat aatat                                            25


<210>  4
<211>  17
<212>  DNA
<213>  Artificial Sequence

<220>
<223>  Vector specific primer for sequencing

<400>  4
caggaaacag ctatgac                                                    17


<210>  5
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  5
ttggggagtt taaggaaa                                                   18


<210>  6
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  6
tggggagttt aaggaaag                                                   18


<210>  7
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  7
ggggagttta aggaaaga                                                   18


<210>  8
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  8
gggagtttaa ggaaagag                                                   18


<210>  9
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  9
ggagtttaag gaaagaga                                                    18


<210>  10
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  10
gagtttaagg aaagagat                                                    18


<210>  11
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  11
agtttaagga aagagatt                                                    18


<210>  12
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  12
gtttaaggaa agagattt                                                    18


<210>  13
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  13
tttaaggaaa gagatttg                                                    18

<210>  14
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  14
ttaaggaaag agatttga                                                    18


<210>  15
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  15
taaggaaaga gatttgat                                                    18


<210>  16
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  16
aaggaaagag atttgatt                                                    18


<210>  17
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  17
aggaaagaga tttgattt                                                    18


<210>  18
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 18
ggaaagagat ttgatttg 18

<210> 19
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 19
gaaagagatt tgatttgg 18

<210> 20
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 20
aaagagattt gatttggt 18

<210> 21
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 21
aagagatttg atttggtt 18

<210> 22
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 22
agagatttga tttggttt 18

<210> 23
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  23
gagatttgat ttggtttt                                                    18


<210>  24
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  24
agatttgatt tggtttty                                                    18


<210>  25
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  25
gatttgattt ggttttyg                                                    18


<210>  26
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  26
atttgatttg gttttygt                                                    18


<210>  27
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  27
tttgatttgg ttttygtt                                                    18


<210>  28
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  28
ttgatttggt tttygttt                                                    18


<210>  29
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  29
tgatttggtt ttygtttt                                                    18


<210>  30
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  30
gatttggttt tygttttg                                                    18


<210>  31
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  31
atttggtttt ygttttgt                                                    18


<210>  32
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  32
tttggtttty gttttgtt                                                    18


<210>  33
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  33
ttggttttyg ttttgttt                                                    18


<210>  34
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  34
tggttttygt tttgtttt                                                    18


<210>  35
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  35
ggttttygtt ttgttttt                                                    18


<210>  36
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  36
gttttygttt tgtttttg                                                    18

<210>  37
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  37
ttttygtttt gtttttga                                                    18


<210>  38
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  38
tttygttttg tttttgat                                                    18


<210>  39
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  39
ttygttttgt ttttgata                                                    18


<210>  40
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  40
tygttttgtt tttgatat                                                    18


<210>  41
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 41
ygttttgttt ttgatatt 18

<210> 42
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 42
gttttgtttt tgatattt 18

<210> 43
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 43
ttttgttttt gatatttt 18

<210> 44
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 44
tttgtttttg atattttt 18

<210> 45
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 45
ttgtttttga tatttttt 18

<210> 46
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  46
tgtttttgat attttttt                                                    18


<210>  47
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  47
gtttttgata tttttttt                                                    18


<210>  48
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  48
tttttgatat tttttttt                                                    18


<210>  49
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  49
ttttgatatt tttttttt                                                    18


<210>  50
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  50
tttgatattt tttttttt                                                    18


<210>  51
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  51
ttgatatttt ttttttta                                                    18


<210>  52
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  52
tgatattttt tttttat                                                     18


<210>  53
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  53
   gatatttttt tttttata                                                     18


<210>  54
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  54
atattttttt ttttataa                                                    18


<210>  55
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  55
tatttttttt tttataag                                                    18


<210>  56
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  56
attttttttt ttataagg                                                    18


<210>  57
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  57
tttttttttt tataaggg                                                    18


<210>  58
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  58
tttttttttt ataagggt                                                    18


<210>  59
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  59
ttttttttta taagggtt                                                    18

<210>  60
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  60
ttttttttat aagggttg                                                    18


<210>  61
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  61
tttttttata agggttga                                                    18


<210>  62
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  62
ttttttataa gggttgag                                                    18


<210>  63
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  63
tttttataag ggttgaga                                                    18


<210>  64
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 64
ttttataagg gttgagag 18

<210> 65
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 65
tttataaggg ttgagaga 18

<210> 66
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 66
ttataagggt tgagagat 18

<210> 67
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 67
tataagggtt gagagatt 18

<210> 68
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 68
ataagggttg agagatta 18

<210> 69
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  69
taagggttga gagattag                                                    18


<210>  70
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  70
aagggttgag agattagg                                                    18


<210>  71
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  71
agggttgaga gattaggt                                                    18


<210>  72
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  72
gggttgagag attaggtt                                                    18


<210>  73
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  73
ggttgagaga ttaggttg                                                    18


<210>  74
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  74
gttgagagat taggttgt                                                    18


<210>  75
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  75
ttgagagatt aggttgtt                                                    18


<210>  76
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  76
tgagagatta ggttgttt                                                    18


<210>  77
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  77
gagagattag gttgtttt                                                    18


<210>  78
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  78
agagattagg ttgttttt                                                    18


<210>  79
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  79
gagattaggt tgtttttt                                                    18


<210>  80
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  80
   agattaggtt gtttttty                                                   18


<210>  81
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  81
gattaggttg ttttttyg                                                    18


<210>  82
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  82
attaggttgt tttttygg                                                    18

<210>  83
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  83
ttaggttgtt ttttyggg                                                    18


<210>  84
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  84
taggttgttt tttyggga                                                    18


<210>  85
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  85
aggttgtttt ttygggat                                                    18


<210>  86
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  86
ggttgttttt tygggatt                                                    18


<210>  87
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 87
gttgtttttt ygggatty 18

<210> 88
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 88
ttgtttttty gggattyg 18

<210> 89
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 89
tgttttttyg ggattygt 18

<210> 90
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 90
gttttttygg gattygtt 18

<210> 91
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 91
ttttttyggg attygttt 18

<210> 92
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  92
tttttyggga ttygtttt                                                    18


<210>  93
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  93
ttttygggat tygttttt                                                    18


<210>  94
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  94
tttygggatt ygtttttt                                                    18


<210>  95
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  95
ttygggatty gttttttt                                                    18


<210>  96
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  96
tygggattyg tttttttt                                                    18


<210>  97
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  97
ygggattygt ttttttty                                                    18


<210>  98
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  98
gggattygtt ttttttyg                                                    18


<210>  99
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  99
ggattygttt tttttygg                                                    18


<210>  100
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  100
gattygtttt ttttyggg                                                    18


<210>  101
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  101
attygttttt tttyggga                                                    18


<210>  102
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  102
ttygtttttt ttygggaa                                                    18


<210>  103
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  103
tygttttttt tygggaaa                                                    18


<210>  104
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  104
ygtttttttt ygggaaay                                                    18


<210>  105
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  105
   gtttttttty gggaaayg                                                    18

<210>  106
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  106
   ttttttttyg ggaaaygy                                                    18


<210>  107
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  107
tttttttygg gaaaygyg                                                   18


<210>  108
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  108
ttttttyggg aaaygyga                                                   18


<210>  109
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  109
tttttyggga aaygygag                                                   18


<210>  110
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 110
ttttygggaa aygygagg 18

<210> 111
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 111
tttygggaaa ygygagga 18

<210> 112
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 112
ttygggaaay gygaggat 18

<210> 113
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 113
tygggaaayg ygaggatg 18

<210> 114
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 114
ygggaaaygy gaggatgt 18

<210> 115
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  115
gggaaaygyg aggatgtt                                                    18


<210>  116
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  116
ggaaaygyga ggatgttt                                                    18


<210>  117
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  117
gaaaygygag gatgtttt                                                    18


<210>  118
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  118
aaaygygagg atgtttta                                                    18


<210>  119
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  119
aaygygagga tgttttat                                                    18


<210>  120
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  120
aygygaggat gttttatg                                                    18


<210>  121
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  121
ygygaggatg ttttatgg                                                    18


<210>  122
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  122
gygaggatgt tttatgga                                                    18


<210>  123
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  123
ygaggatgtt ttatggag                                                    18


<210>  124
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  124
gaggatgttt tatggagy                                                    18


<210>  125
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  125
aggatgtttt atggagyg                                                    18


<210>  126
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  126
ggatgtttta tggagygt                                                    18


<210>  127
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  127
gatgttttat ggagygtg                                                    18


<210>  128
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  128
atgttttatg gagygtga                                                    18

<210>  129
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  129
tgttttatgg agygtgag                                                    18


<210>  130
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  130
gttttatgga gygtgagt                                                    18


<210>  131
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  131
ttttatggag ygtgagta                                                    18


<210>  132
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  132
tttatggagy gtgagtat                                                    18


<210>  133
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 133
ttatggagyg tgagtatt 18

<210> 134
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 134
tatggagygt gagtattt 18

<210> 135
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 135
atggagygtg agtattta 18

<210> 136
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 136
tggagygtga gtatttaa 18

<210> 137
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 137
ggagygtgag tatttaat 18

<210> 138
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  138
gagygtgagt atttaatt                                                    18


<210>  139
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  139
agygtgagta tttaattt                                                    18


<210>  140
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  140
gygtgagtat ttaatttt                                                    18


<210>  141
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  141
ygtgagtatt taattttt                                                    18


<210>  142
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  142
gtgagtattt aatttttt                                                    18


<210>  143
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  143
tgagtattta attttttt                                                    18


<210>  144
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  144
gagtatttaa tttttttt                                                    18


<210>  145
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  145
agtatttaat tttttttt                                                    18


<210>  146
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  146
gtatttaatt tttttttt                                                    18


<210>  147
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  147
tatttaattt ttttttta                                                    18


<210>  148
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  148
   atttaatttt ttttttat                                                     18


<210>  149
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  149
tttaattttt tttttata                                                    18


<210>  150
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  150
ttaatttttt ttttatat                                                    18


<210>  151
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  151
taattttttt tttatata                                                    18

<210>  152
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  152
aatttttttt ttatataa                                                    18


<210>  153
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  153
attttttttt tatataaa                                                    18


<210>  154
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  154
tttttttttt atataaaa                                                    18


<210>  155
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  155
ttttttttta tataaaat                                                    18


<210>  156
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 156
ttttttttat ataaaatt 18

<210> 157
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 157
tttttttata taaaattt 18

<210> 158
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 158
ttttttatat aaaatttg 18

<210> 159
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 159
tttttatata aaatttgt 18

<210> 160
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 160
ttttatataa aatttgtt 18

<210> 161
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  161
tttatataaa atttgttt                                                    18


<210>  162
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  162
ttatataaaa tttgtttg                                                    18


<210>  163
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  163
tatataaaat ttgtttgt                                                    18


<210>  164
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  164
atataaaatt tgtttgtt                                                    18


<210>  165
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  165
tataaaattt gtttgtty                                                    18


<210>  166
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  166
ataaaatttg tttgttyg                                                    18


<210>  167
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  167
taaaatttgt ttgttygt                                                    18


<210>  168
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  168
aaaatttgtt tgttygtt                                                    18


<210>  169
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  169
aaatttgttt gttygttt                                                    18


<210>  170
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  170
aatttgtttg ttygtttt                                                    18


<210>  171
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  171
atttgtttgt tygttttt                                                    18


<210>  172
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  172
tttgtttgtt ygtttttt                                                    18


<210>  173
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  173
ttgtttgtty gttttttt                                                    18


<210>  174
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  174
tgtttgttyg tttttttg                                                    18

<210>  175
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  175
   gtttgttygt ttttttgg                                                    18


<210>  176
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  176
tttgttygtt tttttggt                                                   18


<210>  177
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  177
ttgttygttt ttttggtt                                                   18


<210>  178
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  178
tgttygtttt tttggttt                                                   18


<210>  179
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 179
gttygttttt ttggtttt 18

<210> 180
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 180
ttygtttttt tggttttt 18

<210> 181
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 181
tygttttttt ggtttttt 18

<210> 182
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 182
   ygtttttttg gttttttt 18

<210> 183
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 183
gtttttttgg tttttttt 18

<210> 184
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  184
tttttttggt tttttttt                                                    18


<210>  185
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  185
ttttttggtt tttttttg                                                    18


<210>  186
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  186
tttttggttt ttttttgt                                                    18


<210>  187
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  187
ttttggtttt tttttgta                                                    18


<210>  188
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  188
tttggttttt ttttgtaa                                                    18


<210>  189
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  189
ttggtttttt tttgtaaa                                                    18


<210>  190
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  190
tggttttttt ttgtaaag                                                    18


<210>  191
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  191
ggtttttttt tgtaaagt                                                    18


<210>  192
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  192
gttttttttt gtaaagta                                                    18


<210>  193
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  193
   tttttttttg taaagtaa                                                    18


<210>  194
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  194
   ttttttttgt aaagtaag                                                    18


<210>  195
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  195
   tttttttgta aagtaagt                                                    18


<210>  196
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  196
   ttttttgtaa agtaagta                                                    18


<210>  197
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  197
   tttttgtaaa gtaagtaa                                                    18

<210>  198
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  198
ttttgtaaag taagtaag                                                    18


<210>  199
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  199
tttgtaaagt aagtaagt                                                    18


<210>  200
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  200
ttgtaaagta agtaagtt                                                    18


<210>  201
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  201
tgtaaagtaa gtaagttg                                                    18


<210>  202
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 202
gtaaagtaag taagttgy 18

<210> 203
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 203
taaagtaagt aagttgyg 18

<210> 204
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 204
aaagtaagta agttgygt 18

<210> 205
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 205
aagtaagtaa gttgygtt 18

<210> 206
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 206
agtaagtaag ttgygttt 18

<210> 207
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  207
gtaagtaagt tgygtttg                                                    18


<210>  208
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  208
taagtaagtt gygtttgg                                                    18


<210>  209
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  209
aagtaagttg ygtttggt                                                    18


<210>  210
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  210
agtaagttgy gtttggta                                                    18


<210>  211
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  211
gtaagttgyg tttggtaa                                                    18


<210>  212
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  212
taagttgygt ttggtaaa                                                    18


<210>  213
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  213
aagttgygtt tggtaaat                                                    18


<210>  214
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  214
agttgygttt ggtaaata                                                    18


<210>  215
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  215
gttgygtttg gtaaataa                                                    18


<210>  216
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  216
ttgygtttgg taaataat                                                    18


<210>  217
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  217
tgygtttggt aaataatg                                                    18


<210>  218
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  218
gygtttggta aataatga                                                    18


<210>  219
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  219
ygtttggtaa ataatgaa                                                    18


<210>  220
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  220
gtttggtaaa taatgaaa                                                    18

<210>  221
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  221
tttggtaaat aatgaaat                                                    18


<210>  222
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  222
ttggtaaata atgaaatg                                                    18


<210>  223
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  223
tggtaaataa tgaaatgg                                                    18


<210>  224
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  224
ggtaaataat gaaatgga                                                    18


<210>  225
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
      gene.

<400>  225
gtaaataatg aaatggaa                                                    18


<210>  226
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  226
taaataatga aatggaag                                                    18


<210>  227
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  227
aaataatgaa atggaagt                                                    18


<210>  228
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  228
aataatgaaa tggaagtg                                                    18


<210>  229
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  229
ataatgaaat ggaagtgt                                                    18


<210>  230
<211>  18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  230
taatgaaatg gaagtgta                                                    18


<210>  231
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  231
aatgaaatgg aagtgtaa                                                    18


<210>  232
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  232
atgaaatgga agtgtaag                                                    18


<210>  233
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  233
tgaaatggaa gtgtaagg                                                    18


<210>  234
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  234
gaaatggaag tgtaagga                                                    18


<210>  235
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  235
aaatggaagt gtaaggag                                                    18


<210>  236
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  236
aatggaagtg taaggagg                                                    18


<210>  237
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  237
atggaagtgt aaggaggt                                                    18


<210>  238
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  238
tggaagtgta aggaggtt                                                    18


<210>  239
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  239
ggaagtgtaa ggaggtta                                                    18


<210>  240
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  240
gaagtgtaag gaggttaa                                                    18


<210>  241
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  241
aagtgtaagg aggttaag                                                    18


<210>  242
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  242
agtgtaagga ggttaagt                                                    18


<210>  243
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  243
gtgtaaggag gttaagtt                                                    18

<210>  244
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  244
tgtaaggagg ttaagtta                                                    18


<210>  245
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  245
gtaaggaggt taagttaa                                                    18


<210>  246
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  246
taaggaggtt aagttaat                                                    18


<210>  247
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  247
aaggaggtta agttaata                                                    18


<210>  248
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 248
aggaggttaa gttaatag 18

<210> 249
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 249
ggaggttaag ttaatagg 18

<210> 250
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 250
gaggttaagt taataggt 18

<210> 251
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 251
aggttaagtt aataggtg 18

<210> 252
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 252
ggttaagtta ataggtgg 18

<210> 253
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  253
gttaagttaa taggtggt                                                    18


<210>  254
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  254
ttaagttaat aggtggta                                                    18


<210>  255
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  255
taagttaata ggtggtaa                                                    18


<210>  256
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  256
aagttaatag gtggtaay                                                    18


<210>  257
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  257
agttaatagg tggtaayg                                                    18


<210>  258
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  258
gttaataggt ggtaaygg                                                    18


<210>  259
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  259
ttaataggtg gtaayggg                                                    18


<210>  260
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  260
taataggtgg taaygggt                                                    18


<210>  261
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  261
aataggtggt aaygggtt                                                    18


<210>  262
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  262
ataggtggta aygggtta                                                    18


<210>  263
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  263
taggtggtaa ygggttaa                                                    18


<210>  264
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  264
aggtggtaay gggttaat                                                    18


<210>  265
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  265
ggtggtaayg ggttaata                                                    18


<210>  266
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  266
gtggtaaygg gttaataa                                                    18

<210>  267
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  267
tggtaayggg ttaataag                                                    18


<210>  268
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  268
ggtaaygggt taataagt                                                    18


<210>  269
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  269
gtaaygggtt aataagtg                                                    18


<210>  270
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  270
taaygggtta ataagtgt                                                    18


<210>  271
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 271
aaygggttaa taagtgtt 18

<210> 272
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 272
aygggttaat aagtgttg 18

<210> 273
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 273
ygggttaata agtgttgg 18

<210> 274
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 274
gggttaataa gtgttggy 18

<210> 275
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 275
ggttaataag tgttggyg 18

<210> 276
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  276
gttaataagt gttggygy                                                    18


<210>  277
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  277
ttaataagtg ttggygyg                                                    18


<210>  278
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  278
taataagtgt tggygygg                                                    18


<210>  279
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  279
aataagtgtt ggygyggg                                                    18


<210>  280
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  280
ataagtgttg gygygggg                                                    18


<210>  281
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  281
taagtgttgg ygyggggt                                                    18


<210>  282
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  282
aagtgttggy gyggggtt                                                    18


<210>  283
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  283
agtgttggyg yggggtty                                                    18


<210>  284
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  284
gtgttggygy ggggttyg                                                    18


<210>  285
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  285
tgttggygyg gggttygt                                                    18


<210>  286
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  286
gttggygygg ggttygtt                                                    18


<210>  287
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  287
ttggygyggg gttygtta                                                    18


<210>  288
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  288
tggygygggg ttygttag                                                    18


<210>  289
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  289
ggygyggggt tygttagg                                                    18

<210>  290
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  290
gygyggggtt ygttaggg                                                    18


<210>  291
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  291
ygyggggtty gttagggt                                                    18


<210>  292
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  292
gyggggttyg ttagggtg                                                    18


<210>  293
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  293
yggggttygt tagggtgg                                                    18


<210>  294
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 294
ggggttygtt agggtgga 18

<210> 295
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 295
gggttygtta gggtggag 18

<210> 296
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 296
ggttygttag ggtggagg 18

<210> 297
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 297
gttygttagg gtggaggt 18

<210> 298
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 298
ttygttaggg tggaggtt 18

<210> 299
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  299
tygttagggt ggaggttg                                                    18


<210>  300
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  300
tygttagggt ggaggttg                                                    18


<210>  301
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  301
ygttagggtg gaggttga                                                    18


<210>  302
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  302
gttagggtgg aggttgag                                                    18


<210>  303
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  303
ttagggtgga ggttgaga                                                    18


<210>  304
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  304
tagggtggag gttgagaa                                                    18


<210>  305
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  305
agggtggagg ttgagaay                                                    18


<210>  306
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  306
gggtggaggt tgagaayg                                                    18


<210>  307
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  307
ggtggaggtt gagaaygt                                                    18


<210>  308
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  308
gtggaggttg agaaygtt                                                    18


<210>  309
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  309
tggaggttga gaaygttt                                                    18


<210>  310
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  310
ggaggttgag aaygtttt                                                    18


<210>  311
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  311
gaggttgaga aygttttt                                                    18


<210>  312
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  312
aggttgagaa ygtttttt                                                    18

<210>  313
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  313
ggttgagaay gtttttty                                                    18


<210>  314
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  314
gttgagaayg ttttttyg                                                    18


<210>  315
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  315
ttgagaaygt tttttygg                                                    18


<210>  316
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  316
tgagaaygtt ttttyggg                                                    18


<210>  317
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 317
gagaaygttt tttygggt 18

<210> 318
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 318
agaaygtttt ttygggtg 18

<210> 319
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 319
gaaygttttt tygggtgg 18

<210> 320
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 320
aaygtttttt ygggtggt 18

<210> 321
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 321
   aygtttttty gggtggtt 18

<210> 322
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  322
ygtttttyg ggtggttg                                                    18


<210>  323
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  323
gtttttygg gtggttgg                                                    18


<210>  324
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  324
ttttttyggg tggttggy                                                   18


<210>  325
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  325
tttttygggt ggttggyg                                                   18


<210>  326
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  326
ttttygggtg gttggygy                                                    18


<210>  327
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  327
tttygggtgg ttggygyg                                                    18


<210>  328
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  328
ttygggtggt tggygygg                                                    18


<210>  329
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  329
tygggtggtt ggygyggg                                                    18


<210>  330
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  330
ygggtggttg gygygggg                                                    18


<210>  331
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  331
gggtggttgg ygyggggt                                                    18


<210>  332
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  332
ggtggttggy gyggggtt                                                    18


<210>  333
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  333
gtggttggyg yggggttg                                                    18


<210>  334
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  334
tggttggygy ggggttgg                                                    18


<210>  335
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  335
ggttggygyg gggttgga                                                    18

<210>  336
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  336
gttggygygg ggttggag                                                    18


<210>  337
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  337
ttggygyggg gttggaga                                                    18


<210>  338
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  338
tggygygggg ttggagay                                                    18


<210>  339
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  339
ggygyggggt tggagayg                                                    18


<210>  340
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 340
gygyggggtt ggagaygg 18

<210> 341
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 341
ygyggggttg gagayggt 18

<210> 342
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 342
gyggggttgg agayggtt 18

<210> 343
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 343
yggggttgga gayggtty 18

<210> 344
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 344
ggggttggag ayggttyg 18

<210> 345
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  345
gggttggaga yggttygy                                                    18


<210>  346
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  346
ggttggagay ggttygyg                                                    18


<210>  347
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  347
gttggagayg gttygyga                                                    18


<210>  348
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  348
ttggagaygg ttygygag                                                    18


<210>  349
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  349
tggagayggt tygygagt                                                    18


<210>  350
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  350
ggagayggtt ygygagtg                                                    18


<210>  351
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  351
gagayggtty gygagtgt                                                    18


<210>  352
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  352
agayggttyg ygagtgtg                                                    18


<210>  353
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  353
gayggttygy gagtgtga                                                    18


<210>  354
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  354
ayggttygyg agtgtgag                                                    18


<210>  355
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  355
yggttygyga gtgtgagy                                                    18


<210>  356
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  356
ggttygygag tgtgagyg                                                    18


<210>  357
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  357
gttygygagt gtgagygg                                                    18


<210>  358
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  358
ttygygagtg tgagyggy                                                    18

<210>  359
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  359
tygygagtgt gagyggyg                                                    18


<210>  360
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  360
ygygagtgtg agyggygt                                                    18


<210>  361
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  361
gygagtgtga gyggygtt                                                    18


<210>  362
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  362
ygagtgtgag yggygttt                                                    18


<210>  363
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 363
gagtgtgagy ggygtttg 18

<210> 364
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 364
agtgtgagyg gygtttgt 18

<210> 365
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 365
gtgtgagygg ygtttgtt 18

<210> 366
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 366
tgtgagyggy gtttgttt 18

<210> 367
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 367
gtgagyggyg tttgttta 18

<210> 368
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  368
tgagyggygt ttgtttag                                                    18


<210>  369
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  369
gagyggygtt tgtttagg                                                    18


<210>  370
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  370
agyggygttt gtttaggg                                                    18


<210>  371
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  371
gyggygtttg tttagggt                                                    18


<210>  372
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  372
yggygtttgt ttagggta                                                    18


<210>  373
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  373
ggygtttgtt tagggtag                                                    18


<210>  374
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  374
gygtttgttt agggtaga                                                    18


<210>  375
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  375
ygtttgttta gggtagat                                                    18


<210>  376
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  376
gtttgtttag ggtagata                                                    18


<210>  377
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  377
tttgtttagg gtagatag                                                    18


<210>  378
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  378
ttgtttaggg tagatagt                                                    18


<210>  379
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  379
tgtttagggt agatagtt                                                    18


<210>  380
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  380
gtttagggta gatagttg                                                    18


<210>  381
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  381
tttagggtag atagttga                                                    18

<210>  382
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  382
ttagggtaga tagttgag                                                    18


<210>  383
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  383
tagggtagat agttgagg                                                    18


<210>  384
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  384
agggtagata gttgaggg                                                    18


<210>  385
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  385
gggtagatag ttgagggy                                                    18


<210>  386
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 386
ggtagatagt tgagggyg 18

<210> 387
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 387
gtagatagtt gagggygg 18

<210> 388
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 388
tagatagttg agggyggg 18

<210> 389
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 389
agatagttga gggygggg 18

<210> 390
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 390
gatagttgag ggyggggg 18

<210> 391
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  391
   atagttgagg gygggggt                                                    18


<210>  392
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  392
tagttgaggg ygggggtg                                                   18


<210>  393
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  393
agttgagggy gggggtgg                                                   18


<210>  394
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  394
gttgagggyg ggggtgga                                                   18


<210>  395
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  395
ttgagggygg gggtggat                                                    18


<210>  396
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  396
tgagggyggg ggtggatg                                                    18


<210>  397
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  397
gagggygggg gtggatgt                                                    18


<210>  398
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  398
agggyggggg tggatgtt                                                    18


<210>  399
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  399
gggygggggt ggatgttg                                                    18


<210>  400
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  400
   ggygggggtg gatgttgg                                                    18


<210>  401
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  401
   gygggggtgg atgttgga                                                    18


<210>  402
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  402
   ygggggtgga tgttggat                                                    18


<210>  403
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  403
   gggggtggat gttggatg                                                    18


<210>  404
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  404
   ggggtggatg ttggatgg                                                    18

<210>  405
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  405
gggtggatgt tggatgga                                                    18


<210>  406
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  406
ggtggatgtt ggatggat                                                    18


<210>  407
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  407
gtggatgttg gatggatt                                                    18


<210>  408
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  408
tggatgttgg atggatta                                                    18


<210>  409
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 409
ggatgttgga tggattag 18

<210> 410
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 410
gatgttggat ggattaga 18

<210> 411
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 411
atgttggatg gattagaa 18

<210> 412
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 412
tgttggatgg attagaat 18

<210> 413
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 413
gttggatgga ttagaatt 18

<210> 414
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  414
ttggatggat tagaatta                                                    18


<210>  415
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  415
tggatggatt agaattat                                                    18


<210>  416
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  416
ggatggatta gaattatt                                                    18


<210>  417
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  417
gatggattag aattatta                                                    18


<210>  418
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  418
atggattaga attattat                                                    18


<210>  419
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  419
tggattagaa ttattata                                                    18


<210>  420
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  420
ggattagaat tattatat                                                    18


<210>  421
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  421
gattagaatt attatatt                                                    18


<210>  422
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  422
attagaatta ttatattt                                                    18


<210>  423
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  423
ttagaattat tatatttg                                                    18


<210>  424
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  424
tagaattatt atatttgg                                                    18


<210>  425
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  425
agaattatta tatttggg                                                    18


<210>  426
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  426
gaattattat atttgggt                                                    18


<210>  427
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  427
aattattata tttgggtt                                                    18

<210>  428
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  428
attattatat ttgggttt                                                    18


<210>  429
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  429
ttattatatt tgggtttg                                                    18


<210>  430
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  430
tattatattt gggtttgt                                                    18


<210>  431
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  431
attatatttg ggtttgtt                                                    18


<210>  432
<211>  18
<212>  DNA
<213>  Artificial Sequence

<220>
<223>  Oligonucleotide with nucleotide sequence identical to and
       specific for bisulfite converted methylated and/or non-methylated

sense strand DNA of the SNCA(-926/-483) region of the human SNCA
gene.

<400> 432
ttatatttgg gtttgttg 18

<210> 433
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 433
tatatttggg tttgttgt 18

<210> 434
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 434
atatttgggt ttgttgtt 18

<210> 435
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 435
tatttgggtt tgttgttt 18

<210> 436
<211> 18
<212> DNA
<213> Artificial Sequence

<220>
<223> Oligonucleotide with nucleotide sequence identical to and
specific for bisulfite converted methylated and/or non-methylated
sense strand DNA of the SNCA(-926/-483) region of the human SNCA
gene.

<400> 436
atttgggttt gttgtttg 18

<210> 437
<211> 18

<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  437
tttgggtttg ttgtttgt                                                    18


<210>  438
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  438
ttgggtttgt tgtttgtt                                                    18


<210>  439
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  439
tgggtttgtt gtttgttt                                                    18


<210>  440
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  440
gggtttgttg tttgtttg                                                    18


<210>  441
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  441
ggtttgttgt ttgtttga                                                    18

<210>  442
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  442
gtttgttgtt tgtttgag                                                    18

<210>  443
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  443
tttgttgttt gtttgagt                                                    18

<210>  444
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  444
ttgttgtttg tttgagtt                                                    18

<210>  445
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  445
tgttgtttgt ttgagttt                                                    18

<210>  446
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  446
gttgtttgtt tgagtttg                                                    18


<210>  447
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  447
ttgtttgttt gagtttga                                                    18


<210>  448
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  448
tgtttgtttg agtttgaa                                                    18


<210>  449
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  449
gtttgtttga gtttgaat                                                    18


<210>  450
<211>  18
<212>  DNA
<213>  Artificial Sequence

<220>
<223>  Oligonucleotide with nucleotide sequence identical to and
      specific for bisulfite converted methylated and/or non-methylated
      sense strand DNA of the SNCA(-926/-483) region of the human SNCA
      gene.

<400>  450
tttgtttgag tttgaatt                                                    18

<210>  451
<211>  18
<212>  DNA
<213>  Artificial Sequence

<220>
<223>  Oligonucleotide with nucleotide sequence identical to and
       specific for bisulfite converted methylated and/or non-methylated
       sense strand DNA of the SNCA(-926/-483) region of the human SNCA
       gene.

<400>  451
ttgtttgagt ttgaatta                                                    18


<210>  452
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  452
taattcaaac tcaaacaa                                                    18


<210>  453
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  453
aattcaaact caaacaaa                                                    18


<210>  454
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  454
attcaaactc aaacaaac                                                    18


<210>  455
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 455
ttcaaactca aacaaaca 18


<210> 456
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 456
tcaaactcaa acaaacaa 18


<210> 457
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 457
caaactcaaa caaacaac 18


<210> 458
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 458
aaactcaaac aaacaaca 18


<210> 459
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 459
aactcaaaca aacaacaa 18


<210> 460
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  460
actcaaacaa acaacaaa                                                    18


<210>  461
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  461
ctcaaacaaa caacaaac                                                    18


<210>  462
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  462
tcaaacaaac aacaaacc                                                    18


<210>  463
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  463
caaacaaaca acaaaccc                                                    18


<210>  464
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  464
aaacaaacaa caaaccca                                                    18


<210>  465
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  465
aacaaacaac aaacccaa                                                    18


<210>  466
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  466
acaaacaaca aacccaaa                                                    18


<210>  467
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  467
caaacaacaa acccaaat                                                    18


<210>  468
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  468
aaacaacaaa cccaaata                                                    18


<210>  469
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  469
aacaacaaac ccaaatat                                                    18


<210>  470
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  470
acaacaaacc caaatata                                                    18


<210>  471
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  471
caacaaaccc aaatataa                                                    18


<210>  472
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  472
aacaaaccca aatataat                                                    18


<210>  473
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  473
acaaacccaa atataata                                                    18

<210>  474
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  474
caaacccaaa tataataa                                                    18


<210>  475
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  475
aaacccaaat ataataat                                                    18


<210>  476
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  476
aacccaaata taataatt                                                    18


<210>  477
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  477
acccaaatat aataattc                                                    18


<210>  478
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 478
cccaaatata ataattct 18

<210> 479
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 479
ccaaatataa taattcta 18

<210> 480
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 480
caaatataat aattctaa 18

<210> 481
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 481
aaatataata attctaat 18

<210> 482
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 482
aatataataa ttctaatc 18

<210> 483
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  483
atataataat tctaatcc                                                    18


<210>  484
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  484
tataataatt ctaatcca                                                    18


<210>  485
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  485
ataataattc taatccat                                                    18


<210>  486
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  486
taataattct aatccatc                                                    18


<210>  487
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  487
aataattcta atccatcc                                                    18


<210>  488
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  488
ataattctaa tccatcca                                                    18


<210>  489
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  489
taattctaat ccatccaa                                                    18


<210>  490
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  490
aattctaatc catccaac                                                    18


<210>  491
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  491
attctaatcc atccaaca                                                    18


<210>  492
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  492
ttctaatcca tccaacat                                                    18


<210>  493
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  493
tctaatccat ccaacatc                                                    18


<210>  494
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  494
ctaatccatc caacatcc                                                    18


<210>  495
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  495
taatccatcc aacatcca                                                    18


<210>  496
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  496
aatccatcca acatccac                                                    18

<210>  497
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  497
atccatccaa catccacc                                                    18


<210>  498
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  498
tccatccaac atccaccc                                                    18


<210>  499
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  499
ccatccaaca tccacccc                                                    18


<210>  500
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  500
catccaacat ccaccccc                                                    18


<210>  501
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 501
atccaacatc cacccccr 18

<210> 502
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 502
tccaacatcc acccccrc 18

<210> 503
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 503
ccaacatcca cccccrcc 18

<210> 504
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 504
caacatccac ccccrccc 18

<210> 505
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 505
aacatccacc cccrccct 18

<210> 506
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  506
acatccaccc ccrccctc                                                    18


<210>  507
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  507
catccacccc crccctca                                                    18


<210>  508
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  508
atccaccccc rccctcaa                                                    18


<210>  509
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  509
   tccacccccr ccctcaac                                                     18


<210>  510
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  510
ccacccccrc cctcaact                                                    18


<210>  511
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  511
cacccccrcc ctcaacta                                                    18


<210>  512
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  512
acccccrccc tcaactat                                                    18


<210>  513
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  513
cccccrccct caactatc                                                    18


<210>  514
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  514
ccccrccctc aactatct                                                    18


<210>  515
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  515
cccrccctca actatcta                                                    18


<210>  516
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  516
ccrccctcaa ctatctac                                                    18


<210>  517
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  517
crccctcaac tatctacc                                                    18


<210>  518
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  518
rccctcaact atctaccc                                                    18


<210>  519
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  519
ccctcaacta tctaccct                                                    18

<210>  520
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  520
cctcaactat ctacccta                                                    18


<210>  521
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  521
ctcaactatc taccctaa                                                    18


<210>  522
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  522
tcaactatct accctaaa                                                    18


<210>  523
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  523
caactatcta ccctaaac                                                    18


<210>  524
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 524
aactatctac cctaaaca 18

<210> 525
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 525
actatctacc ctaaacaa 18

<210> 526
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 526
ctatctaccc taaacaaa 18

<210> 527
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 527
tatctaccct aaacaaac 18

<210> 528
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 528
atctacccta aacaaacr 18

<210> 529
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  529
tctaccctaa acaaacrc                                                    18


<210>  530
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  530
ctaccctaaa caaacrcc                                                    18


<210>  531
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  531
taccctaaac aaacrccr                                                    18


<210>  532
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  532
accctaaaca aacrccrc                                                    18


<210>  533
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  533
ccctaaacaa acrccrct                                                    18


<210>  534
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  534
cctaaacaaa crccrctc                                                    18


<210>  535
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  535
ctaaacaaac rccrctca                                                    18


<210>  536
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  536
taaacaaacr ccrctcac                                                    18


<210>  537
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  537
aaacaaacrc crctcaca                                                    18


<210>  538
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  538
aacaaacrcc rctcacac                                                    18


<210>  539
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  539
acaaacrccr ctcacact                                                    18


<210>  540
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  540
caaacrccrc tcacactc                                                    18


<210>  541
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  541
aaacrccrct cacactcr                                                    18


<210>  542
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  542
aacrccrctc acactcrc                                                    18

<210>  543
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  543
acrccrctca cactcrcr                                                    18


<210>  544
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  544
crccrctcac actcrcra                                                    18


<210>  545
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  545
rccrctcaca ctcrcraa                                                    18


<210>  546
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  546
ccrctcacac tcrcraac                                                    18


<210>  547
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 547
crctcacact crcraacc 18

<210> 548
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 548
rctcacactc rcraaccr 18

<210> 549
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 549
ctcacactcr craaccrt 18

<210> 550
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 550
tcacactcrc raaccrtc 18

<210> 551
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 551
cacactcrcr aaccrtct 18

<210> 552
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  552
acactcrcra accrtctc                                                     18


<210>  553
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  553
cactcrcraa ccrtctcc                                                     18


<210>  554
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  554
actcrcraac crtctcca                                                     18


<210>  555
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  555
ctcrcraacc rtctccaa                                                     18


<210>  556
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  556
tcrcraaccr tctccaac                                                    18


<210>  557
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  557
crcraaccrt ctccaacc                                                    18


<210>  558
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  558
rcraaccrtc tccaaccc                                                    18


<210>  559
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  559
craaccrtct ccaacccc                                                    18


<210>  560
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  560
raaccrtctc caaccccr                                                    18


<210>  561
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  561
aaccrtctcc aaccccrc                                                    18


<210>  562
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  562
accrtctcca accccrcr                                                    18


<210>  563
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  563
ccrtctccaa ccccrcrc                                                    18


<210>  564
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  564
crtctccaac cccrcrcc                                                    18


<210>  565
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  565
rtctccaacc ccrcrcca                                                    18

<210>  566
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  566
tctccaaccc crcrccaa                                                    18


<210>  567
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  567
ctccaacccc rcrccaac                                                    18


<210>  568
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  568
tccaaccccr crccaacc                                                    18


<210>  569
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  569
ccaaccccrc rccaacca                                                    18


<210>  570
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400>  570
caaccccrcr ccaaccac                                                    18


<210>  571
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  571
aaccccrcrc caaccacc                                                    18


<210>  572
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  572
accccrcrcc aaccaccc                                                    18


<210>  573
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  573
ccccrcrcca accacccr                                                    18


<210>  574
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  574
cccrcrccaa ccacccra                                                    18


<210>  575
<211>  18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  575
ccrcrccaac cacccraa                                                     18


<210>  576
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  576
crcrccaacc acccraaa                                                     18


<210>  577
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  577
rcrccaacca cccraaaa                                                     18


<210>  578
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  578
crccaaccac ccraaaaa                                                     18


<210>  579
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  579
rccaaccacc craaaaaa                                                    18


<210>  580
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  580
ccaaccaccc raaaaaac                                                    18


<210>  581
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  581
caaccacccr aaaaaacr                                                    18


<210>  582
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  582
aaccacccra aaaaacrt                                                    18


<210>  583
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  583
accacccraa aaaacrtt                                                    18


<210>  584
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  584
ccacccraaa aaacrttc                                                    18


<210>  585
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  585
cacccraaaa aacrttct                                                    18


<210>  586
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  586
acccraaaaa acrttctc                                                    18


<210>  587
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  587
cccraaaaaa crttctca                                                    18


<210>  588
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  588
ccraaaaaac rttctcaa                                                    18

<210>  589
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  589
craaaaaacr ttctcaac                                                    18


<210>  590
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  590
raaaaaacrt tctcaacc                                                    18


<210>  591
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  591
aaaaaacrtt ctcaacct                                                    18


<210>  592
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  592
aaaaacrttc tcaacctc                                                    18


<210>  593
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400>  593
aaaacrttct caacctcc                                                    18


<210>  594
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  594
aaacrttctc aacctcca                                                    18


<210>  595
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  595
aacrttctca acctccac                                                    18


<210>  596
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  596
acrttctcaa cctccacc                                                    18


<210>  597
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  597
crttctcaac ctccaccc                                                    18


<210>  598
<211>  18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  598
rttctcaacc tccaccct                                                    18


<210>  599
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  599
ttctcaacct ccacccta                                                    18


<210>  600
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  600
tctcaacctc caccctaa                                                    18


<210>  601
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  601
ctcaacctcc accctaac                                                    18


<210>  602
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  602
tcaacctcca ccctaacr                                                    18


<210>  603
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  603
caacctccac cctaacra                                                    18


<210>  604
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  604
aacctccacc ctaacraa                                                    18


<210>  605
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  605
acctccaccc taacraac                                                    18


<210>  606
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  606
cctccaccct aacraacc                                                    18


<210>  607
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  607
ctccacccta acraaccc                                                    18


<210>  608
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  608
tccaccctaa craacccc                                                    18


<210>  609
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  609
ccaccctaac raaccccr                                                    18


<210>  610
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  610
caccctaacr aaccccrc                                                    18


<210>  611
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  611
accctaacra accccrcr                                                    18

<210>  612
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  612
ccctaacraa ccccrcrc                                                    18


<210>  613
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  613
cctaacraac cccrcrcc                                                    18


<210>  614
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  614
ctaacraacc ccrcrcca                                                    18


<210>  615
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  615
taacraaccc crcrccaa                                                    18


<210>  616
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 616
aacraacccc rcrccaac 18

<210> 617
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 617
acraaccccr crccaaca 18

<210> 618
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 618
craaccccrc rccaacac 18

<210> 619
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 619
raaccccrcr ccaacact 18

<210> 620
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 620
aaccccrcrc caacactt 18

<210> 621
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  621
accccrcrcc aacactta                                                    18


<210>  622
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  622
ccccrcrcca acacttat                                                    18


<210>  623
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  623
cccrcrccaa cacttatt                                                    18


<210>  624
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  624
ccrcrccaac acttatta                                                    18


<210>  625
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  625
crcrccaaca cttattaa                                                    18


<210>  626
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  626
rcrccaacac ttattaac                                                    18


<210>  627
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  627
crccaacact tattaacc                                                    18


<210>  628
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  628
rccaacactt attaaccc                                                    18


<210>  629
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  629
ccaacactta ttaacccr                                                    18


<210>  630
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  630
caacacttat taacccrt                                                    18


<210>  631
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  631
aacacttatt aacccrtt                                                    18


<210>  632
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  632
acacttatta acccrtta                                                    18


<210>  633
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  633
cacttattaa cccrttac                                                    18


<210>  634
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  634
acttattaac ccrttacc                                                    18

<210>  635
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  635
cttattaacc crttacca                                                    18


<210>  636
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  636
ttattaaccc rttaccac                                                    18


<210>  637
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  637
tattaacccr ttaccacc                                                    18


<210>  638
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  638
attaacccrt taccacct                                                    18


<210>  639
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 639
ttaacccrtt accaccta 18

<210> 640
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 640
taacccrtta ccacctat 18

<210> 641
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 641
aacccrttac cacctatt 18

<210> 642
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 642
acccrttacc acctatta 18

<210> 643
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 643
cccrttacca cctattaa 18

<210> 644
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  644
ccrttaccac ctattaac                                                    18


<210>  645
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  645
crttaccacc tattaact                                                    18


<210>  646
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  646
rttaccacct attaactt                                                    18


<210>  647
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  647
ttaccaccta ttaactta                                                    18


<210>  648
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  648
taccacctat taacttaa                                                    18


<210>  649
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  649
accacctatt aacttaac                                                    18


<210>  650
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  650
ccacctatta acttaacc                                                    18


<210>  651
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  651
cacctattaa cttaacct                                                    18


<210>  652
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  652
acctattaac ttaacctc                                                    18


<210>  653
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  653
cctattaact taacctcc                                                    18


<210>  654
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  654
ctattaactt aacctcct                                                    18


<210>  655
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  655
tattaactta acctcctt                                                    18


<210>  656
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  656
attaacttaa cctcctta                                                    18


<210>  657
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  657
ttaacttaac ctccttac                                                    18

<210>  658
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  658
taacttaacc tccttaca                                                    18


<210>  659
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  659
aacttaacct ccttacac                                                    18


<210>  660
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  660
acttaacctc cttacact                                                    18


<210>  661
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  661
cttaacctcc ttacactt                                                    18


<210>  662
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 662
ttaacctcct tacacttc 18


<210> 663
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 663
taacctcctt acacttcc 18


<210> 664
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 664
aacctcctta cacttcca 18


<210> 665
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 665
acctccttac acttccat 18


<210> 666
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 666
cctccttaca cttccatt 18


<210> 667
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  667
ctccttacac ttccattt                                                    18


<210>  668
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  668
tccttacact tccatttc                                                    18


<210>  669
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  669
ccttacactt ccatttca                                                    18


<210>  670
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  670
cttacacttc catttcat                                                    18


<210>  671
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  671
ttacacttcc atttcatt                                                    18


<210>  672
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  672
tacacttcca tttcatta                                                    18


<210>  673
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  673
acacttccat ttcattat                                                    18


<210>  674
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  674
cacttccatt tcattatt                                                    18


<210>  675
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  675
acttccattt cattattt                                                    18


<210>  676
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  676
cttccatttc attattta                                                    18


<210>  677
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  677
ttccatttca ttatttac                                                    18


<210>  678
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  678
tccatttcat tatttacc                                                    18


<210>  679
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  679
ccatttcatt atttacca                                                    18


<210>  680
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  680
catttcatta tttaccaa                                                    18

<210>  681
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  681
atttcattat ttaccaaa                                                    18


<210>  682
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  682
tttcattatt taccaaac                                                    18


<210>  683
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  683
ttcattattt accaaacr                                                    18


<210>  684
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  684
tcattattta ccaaacrc                                                    18


<210>  685
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 685
cattatttac caaacrca 18

<210> 686
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 686
attatttacc aaacrcaa 18

<210> 687
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 687
ttatttacca aacrcaac 18

<210> 688
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 688
tatttaccaa acrcaact 18

<210> 689
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 689
atttaccaaa crcaactt 18

<210> 690
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  690
tttaccaaac rcaactta                                                    18


<210>  691
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  691
ttaccaaacr caacttac                                                    18


<210>  692
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  692
taccaaacrc aacttact                                                    18


<210>  693
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  693
accaaacrca acttactt                                                    18


<210>  694
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  694
ccaaacrcaa cttactta                                                    18


<210>  695
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  695
caaacrcaac ttacttac                                                    18


<210>  696
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  696
aaacrcaact tacttact                                                    18


<210>  697
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  697
aacrcaactt acttactt                                                    18


<210>  698
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  698
acrcaactta cttacttt                                                    18


<210>  699
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  699
crcaacttac ttacttta                                                    18


<210>  700
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  700
rcaacttact tactttac                                                    18


<210>  701
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  701
caacttactt actttaca                                                    18


<210>  702
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  702
aacttactta ctttacaa                                                    18


<210>  703
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  703
acttacttac tttacaaa                                                    18

<210>  704
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  704
cttacttact ttacaaaa                                                    18


<210>  705
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  705
ttacttactt tacaaaaa                                                    18


<210>  706
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  706
tacttacttt acaaaaaa                                                    18


<210>  707
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  707
acttacttta caaaaaaa                                                    18


<210>  708
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 708
cttactttac aaaaaaaa 18

<210> 709
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 709
ttactttaca aaaaaaaa 18

<210> 710
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 710
tactttacaa aaaaaaac 18

<210> 711
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 711
actttacaaa aaaaaacc 18

<210> 712
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 712
ctttacaaaa aaaaacca 18

<210> 713
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  713
tttacaaaaa aaaaccaa                                                    18


<210>  714
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  714
ttacaaaaaa aaaccaaa                                                    18


<210>  715
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  715
tacaaaaaaa aaccaaaa                                                    18


<210>  716
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  716
acaaaaaaaa accaaaaa                                                    18


<210>  717
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  717
caaaaaaaaa ccaaaaaa                                                    18


<210>  718
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  718
aaaaaaaaac caaaaaaa                                                    18


<210>  719
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  719
aaaaaaaacc aaaaaaac                                                    18


<210>  720
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  720
aaaaaaacca aaaaaacr                                                    18


<210>  721
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  721
aaaaaaccaa aaaaacra                                                    18


<210>  722
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  722
aaaaaccaaa aaaacraa                                                    18


<210>  723
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  723
aaaaccaaaa aaacraac                                                    18


<210>  724
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  724
aaaccaaaaa aacraaca                                                    18


<210>  725
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  725
aaccaaaaaa acraacaa                                                    18


<210>  726
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  726
accaaaaaaa craacaaa                                                    18

<210>  727
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  727
ccaaaaaaac raacaaac                                                    18


<210>  728
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  728
caaaaaaacr aacaaaca                                                    18


<210>  729
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  729
aaaaaaacra acaaacaa                                                    18


<210>  730
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  730
aaaaaacraa caaacaaa                                                    18


<210>  731
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 731
aaaaacraac aaacaaat 18


<210> 732
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 732
aaaacraaca aacaaatt 18


<210> 733
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 733
aaacraacaa acaaattt 18


<210> 734
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 734
aacraacaaa caaatttt 18


<210> 735
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 735
acraacaaac aaatttta 18


<210> 736
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  736
craacaaaca aattttat                                                    18


<210>  737
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  737
raacaaacaa attttata                                                    18


<210>  738
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  738
aacaaacaaa ttttatat                                                    18


<210>  739
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  739
acaaacaaat tttatata                                                    18


<210>  740
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  740
caaacaaatt ttatataa                                                    18


<210>  741
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  741
aaacaaattt tatataaa                                                    18


<210>  742
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  742
aacaaatttt atataaaa                                                    18


<210>  743
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  743
acaaatttta tataaaaa                                                    18


<210>  744
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  744
caaattttat ataaaaaa                                                    18


<210>  745
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  745
aaattttata taaaaaaa                                                    18


<210>  746
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  746
aattttatat aaaaaaaa                                                    18


<210>  747
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  747
attttatata aaaaaaaa                                                    18


<210>  748
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  748
ttttatataa aaaaaaaa                                                    18


<210>  749
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  749
tttatataaa aaaaaaat                                                    18

<210>  750
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  750
ttatataaaa aaaaaatt                                                    18


<210>  751
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  751
tatataaaaa aaaaatta                                                    18


<210>  752
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  752
atataaaaaa aaaattaa                                                    18


<210>  753
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  753
tataaaaaaa aaattaaa                                                    18


<210>  754
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 754
ataaaaaaaa aattaaat 18

<210> 755
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 755
taaaaaaaaa attaaata 18

<210> 756
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 756
aaaaaaaaaa ttaaatac 18

<210> 757
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 757
aaaaaaaaat taaatact 18

<210> 758
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 758
aaaaaaaatt aaatactc 18

<210> 759
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  759
aaaaaaatta aatactca                                                    18


<210>  760
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  760
aaaaaattaa atactcac                                                    18


<210>  761
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  761
aaaaattaaa tactcacr                                                    18


<210>  762
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  762
aaaattaaat actcacrc                                                    18


<210>  763
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  763
aaattaaata ctcacrct                                                    18


<210>  764
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  764
aattaaatac tcacrctc                                                    18


<210>  765
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  765
attaaatact cacrctcc                                                    18


<210>  766
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  766
ttaaatactc acrctcca                                                    18


<210>  767
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  767
taaatactca crctccat                                                    18


<210>  768
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  768
taaatactca crctccat                                                    18


<210>  769
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  769
aaatactcac rctccata                                                    18


<210>  770
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  770
aatactcacr ctccataa                                                    18


<210>  771
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  771
atactcacrc tccataaa                                                    18


<210>  772
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  772
tactcacrct ccataaaa                                                    18

<210>  773
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  773
actcacrctc cataaaac                                                    18


<210>  774
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  774
ctcacrctcc ataaaaca                                                    18


<210>  775
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  775
tcacrctcca taaaacat                                                    18


<210>  776
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  776
cacrctccat aaaacatc                                                    18


<210>  777
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 777
acrctccata aaacatcc 18

<210> 778
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 778
crctccataa aacatcct 18

<210> 779
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 779
rctccataaa acatcctc 18

<210> 780
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 780
ctccataaaa catcctcr 18

<210> 781
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 781
tccataaaac atcctcrc 18

<210> 782
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  782
ccataaaaca tcctcrcr                                                    18


<210>  783
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  783
cataaaacat cctcrcrt                                                    18


<210>  784
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  784
ataaaacatc ctcrcrtt                                                    18


<210>  785
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  785
taaaacatcc tcrcrttt                                                    18


<210>  786
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  786
aaaacatcct crcrtttc                                                    18


<210>  787
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  787
aaacatcctc rcrtttcc                                                    18


<210>  788
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  788
aacatcctcr crtttccc                                                    18


<210>  789
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  789
acatcctcrc rtttcccr                                                    18


<210>  790
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  790
catcctcrcr tttcccra                                                    18


<210>  791
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  791
atcctcrcrt ttcccraa                                                    18


<210>  792
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  792
tcctcrcrtt tcccraaa                                                    18


<210>  793
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  793
cctcrcrttt cccraaaa                                                    18


<210>  794
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  794
ctcrcrtttc ccraaaaa                                                    18


<210>  795
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  795
tcrcrtttcc craaaaaa                                                    18

<210>  796
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  796
crcrtttccc raaaaaaa                                                    18


<210>  797
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  797
rcrtttcccr aaaaaaaa                                                    18


<210>  798
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  798
crtttcccra aaaaaaac                                                    18


<210>  799
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  799
rtttcccraa aaaaaacr                                                    18


<210>  800
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 800
tttcccraaa aaaaacra 18

<210> 801
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 801
ttcccraaaa aaaacraa 18

<210> 802
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 802
tcccraaaaa aaacraat 18

<210> 803
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 803
cccraaaaaa aacraatc 18

<210> 804
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 804
ccraaaaaaa acraatcc 18

<210> 805
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  805
craaaaaaaa craatccc                                                    18


<210>  806
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  806
raaaaaaaac raatcccr                                                    18


<210>  807
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  807
aaaaaaaacr aatcccra                                                    18


<210>  808
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  808
aaaaaaacra atcccraa                                                    18


<210>  809
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  809
aaaaaacraa tcccraaa                                                    18


<210>  810
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  810
aaaaacraat cccraaaa                                                    18


<210>  811
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  811
aaaacraatc ccraaaaa                                                    18


<210>  812
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  812
aaacraatcc craaaaaa                                                    18


<210>  813
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  813
aacraatccc raaaaaac                                                    18


<210>  814
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  814
acraatcccr aaaaaaca                                                    18


<210>  815
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  815
craatcccra aaaaacaa                                                    18


<210>  816
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  816
raatcccraa aaaacaac                                                    18


<210>  817
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  817
aatcccraaa aaacaacc                                                    18


<210>  818
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  818
atcccraaaa aacaacct                                                    18

<210>  819
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  819
tcccraaaaa acaaccta                                                    18


<210>  820
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  820
cccraaaaaa caacctaa                                                    18


<210>  821
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  821
ccraaaaaac aacctaat                                                    18


<210>  822
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  822
craaaaaaca acctaatc                                                    18


<210>  823
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 823
raaaaaacaa cctaatct 18


<210> 824
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 824
aaaaaacaac ctaatctc 18


<210> 825
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 825
aaaaacaacc taatctct 18


<210> 826
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 826
aaaacaacct aatctctc 18


<210> 827
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 827
aaacaaccta atctctca 18


<210> 828
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  828
aacaacctaa tctctcaa                                                    18


<210>  829
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  829
acaacctaat ctctcaac                                                    18


<210>  830
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  830
caacctaatc tctcaacc                                                    18


<210>  831
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  831
aacctaatct ctcaaccc                                                    18


<210>  832
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  832
acctaatctc tcaaccct                                                    18


<210>  833
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  833
cctaatctct caaccctt                                                    18


<210>  834
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  834
ctaatctctc aaccctta                                                    18


<210>  835
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  835
taatctctca acccttat                                                    18


<210>  836
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  836
aatctctcaa cccttata                                                    18


<210>  837
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  837
atctctcaac ccttataa                                                    18


<210>  838
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  838
tctctcaacc cttataaa                                                    18


<210>  839
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  839
ctctcaaccc ttataaaa                                                    18


<210>  840
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  840
tctcaaccct tataaaaa                                                    18


<210>  841
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  841
ctcaaccctt ataaaaaa                                                    18

<210>  842
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  842
tcaaccctta taaaaaaa                                                    18


<210>  843
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  843
caacccttat aaaaaaaa                                                    18


<210>  844
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  844
aacccttata aaaaaaaa                                                    18


<210>  845
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  845
acccttataa aaaaaaaa                                                    18


<210>  846
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 846
cccttataaa aaaaaaaa 18

<210> 847
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 847
ccttataaaa aaaaaaat 18

<210> 848
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 848
cttataaaaa aaaaaata 18

<210> 849
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 849
ttataaaaaa aaaaatat 18

<210> 850
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 850
tataaaaaaa aaaatatc 18

<210> 851
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  851
ataaaaaaaa aaatatca                                                    18


<210>  852
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  852
taaaaaaaaa aatatcaa                                                    18


<210>  853
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  853
aaaaaaaaaa atatcaaa                                                    18


<210>  854
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  854
aaaaaaaaaa tatcaaaa                                                    18


<210>  855
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  855
aaaaaaaaat atcaaaaa                                                    18


<210>  856
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  856
aaaaaaaata tcaaaaac                                                    18


<210>  857
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  857
aaaaaaatat caaaaaca                                                    18


<210>  858
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  858
aaaaaatatc aaaaacaa                                                    18


<210>  859
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  859
aaaaatatca aaaacaaa                                                    18


<210>  860
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  860
aaaatatcaa aaacaaaa                                                    18


<210>  861
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  861
aaatatcaaa aacaaaac                                                    18


<210>  862
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  862
aatatcaaaa acaaaacr                                                    18


<210>  863
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  863
atatcaaaaa caaaacra                                                    18


<210>  864
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  864
tatcaaaaac aaaacraa                                                    18

<210>  865
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  865
atcaaaaaca aaacraaa                                                    18


<210>  866
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  866
tcaaaaacaa aacraaaa                                                    18


<210>  867
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  867
caaaaacaaa acraaaac                                                    18


<210>  868
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  868
aaaaacaaaa craaaacc                                                    18


<210>  869
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 869
aaaacaaaac raaaacca 18

<210> 870
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 870
aaacaaaacr aaaaccaa 18

<210> 871
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 871
aacaaaacra aaaccaaa 18

<210> 872
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 872
acaaaacraa aaccaaat 18

<210> 873
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 873
caaaacraaa accaaatc 18

<210> 874
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  874
aaaacraaaa ccaaatca                                                    18


<210>  875
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  875
aaacraaaac caaatcaa                                                    18


<210>  876
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  876
aacraaaacc aaatcaaa                                                    18


<210>  877
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  877
acraaaacca aatcaaat                                                    18


<210>  878
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  878
craaaaccaa atcaaatc                                                    18


<210>  879
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  879
raaaaccaaa tcaaatct                                                    18


<210>  880
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  880
aaaaccaaat caaatctc                                                    18


<210>  881
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  881
aaaccaaatc aaatctct                                                    18


<210>  882
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  882
aaccaaatca aatctctt                                                    18


<210>  883
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  883
accaaatcaa atctcttt                                                    18


<210>  884
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  884
ccaaatcaaa tctctttc                                                    18


<210>  885
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  885
caaatcaaat ctctttcc                                                    18


<210>  886
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  886
aaatcaaatc tctttcct                                                    18


<210>  887
<211>  18
<212>  DNA
<213>  Artificial Sequence

<220>
<223>  Oligonucleotide with nucleotide sequence specific for bisulfite
      converted methylated and/or non-methylated DNA of the
      SNCA(-926/-483) region of the human SNCA gene, and identical to
      antisense strand.

<400>  887
aatcaaatct ctttcctt                                                    18

<210>  888
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  888
atcaaatctc tttcctta                                                    18


<210>  889
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  889
tcaaatctct ttccttaa                                                    18


<210>  890
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  890
caaatctctt tccttaaa                                                    18


<210>  891
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  891
aaatctcttt ccttaaac                                                    18


<210>  892
<211>  18
<212>  DNA
<213>  Artificial Sequence

<220>
<223>  Oligonucleotide with nucleotide sequence specific for bisulfite
       converted methylated and/or non-methylated DNA of the

SNCA(-926/-483) region of the human SNCA gene, and identical to
antisense strand.

<400> 892
aatctctttc cttaaact 18

<210> 893
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 893
atctctttcc ttaaactc 18

<210> 894
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 894
tctctttcct taaactcc 18

<210> 895
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 895
ctctttcctt aaactccc 18

<210> 896
<211> 18
<212> DNA
<213> Artificial Sequence

<220>
<223> Oligonucleotide with nucleotide sequence specific for bisulfite
converted methylated and/or non-methylated DNA of the
SNCA(-926/-483) region of the human SNCA gene, and identical to
antisense strand.

<400> 896
tctttcctta aactcccc 18

<210> 897
<211> 18

<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  897
ctttccttaa actcccca                                                    18


<210>  898
<211>  18
<212>  DNA
<213>  Artificial Sequence

<220>
<223>  Oligonucleotide with nucleotide sequence specific for bisulfite
       converted methylated and/or non-methylated DNA of the
       SNCA(-926/-483) region of the human SNCA gene, and identical to
       antisense strand.

<400>  898
tttccttaaa ctccccaa                                                    18

## Description

The present invention pertains to the diagnosis of Parkinson's disease, especially to the diagnosis of sporadic Parkinson's disease. The present invention concerns the use of peripheral blood monocytes for determining the methylation status of the human alpha-synuclein gene for diagnosing Parkinson's disease and/or for assessing a patient's risk of developing Parkinson's disease. The present invention further concerns a method for determining methylation of the human alpha-synuclein gene in peripheral blood monocytes for diagnosing Parkinson's disease and/or for assessing a patient's risk of developing Parkinson's disease. Moreover, the present invention comprises a method for diagnosing Parkinson's disease and/or for assessing a patient's risk of developing Parkinson's disease

Parkinson's disease (PD) is one of the most common neurodegenerative diseases. Approximately 2% of elder persons (older than 65) are affected. The clinical symptoms of PD are characterized by akinetic-rigid motor dysfunction and cognitive, affective and neurobehavioural impairments. The most prominent neuropathological characteristics of Parkinson's disease are progressive cell death within the nigro-striatal dopaminergic system, and the appearance of cytoplasmic protein aggregates, the so-called Lewy bodies, in cells of the central nervous system of affected persons.

The majority of PD cases occur sporadically, and its etiology is largely unknown. Pursuant to recent findings, PD is usually diagnosed in an advanced stage of the disease. Therefore, a neuro-protective or neuro-restorative therapy can only be launched late in the disease's progression. Current methods of diagnosing PD, for example single photon emission computed tomography (SPECT) or positron emission tomography (PET), are expensive and are therefore only used subsequent to clinical presumption diagnosis or for differential diagnosis. Although some symptoms such as sensory and sleep difficulties are discussed as useful in early diagnosis of PD, they are not specific enough and lack sensitivity. A simple to perform and inexpensive test for determining the risk of being affected by PD is not available yet.

According to its pathophysiology, PD may be considered a synucleinopathy due to the abnormal accumulation of the alpha-synuclein (SNCA) protein in the brain of patients having developed PD. Since the alpha-synuclein protein is the main component of Lewy bodies, a number of specific genetic mutations of the alpha-synuclein gene associated with PD have been discovered. Missense mutations (mutation in which a single nucleotide is changed) of the gene, and duplications and triplications of the locus containing it have been found in different groups with familial PD. Missense mutations are very rare with few families found in all the world. On the other hand multiplications of the SNCA locus account for around 2% of familial cases. Additionally multiplications have been found in non symptomatic carriers leading to the conclusion that penetrance is incomplete or age-dependent.

A fragment of the alpha-synuclein is known to be the non-Abeta component of Alzheimer's disease amyloid. Said fragment was originally found in an amyloid-enriched fraction, and identified as fragment of its precursor protein, NCAP, by cloning of the full-length cDNA. It was later determined that NCAP is the human homologue of *Torpedo* synuclein. Therefore, NCAP is now referred to as human alpha-synuclein.

It recently became evident that SNCA expression is an important factor in pathogenesis of PD as increased gene dosage of SNCA leads to familial PD, and polymorphisms in the SNCA promoter increase the risk of developing sporadic PD. Furthermore, SNCA mRNA levels were found to be increased in individual laser-captured dopaminergic neurons in substantia nigra of PD patients. It seems that already a minor increase of wild-type SNCA protein in the presynaptic compartment of neurons is sufficient to induce dysfunction and degeneration.

Voustinas and colleagues have studied the expression of alleles of the alpha-synuclein gene in a lymphoblastoid cell line and in blood cells of a patient heterozygous for the mutation p.Ala53Thr, the first mutation implicated in PD pathogenesis (Voustinas, G.E. et al. (2010) Allelic imbalance of expression and epigenetic regulation within the alpha-synuclein wild-type and p.Ala53Thr alleles in Parkinson disease. Hum. Mutat. 31(6):685-691). Said patient was diagnosed with early-onset PD, and belonging to a family with multiple PD cases in several generations, with autosomal dominant inheritance and <100% penetrance of the disease. Evidence is provided that SNCA shows monoalleleic expression in this patient, that epigenetic silencing of the mutated allele involves histone modifications but not DNA methylation, because expression of the mutant allele was silenced despite the finding that all analyzed CpG sites were found to be unmethylated, and that steady state mRNA levels deriving from the normal SNCA allele in this patient exceeded those of both normal SNCA alleles in matching control individuals. An imbalanced SNCA expression has thus been documented, with silencing the expression of the p.Ala53Thr allele and upregulation of the expression of the wild-type allele.

Gründemann and colleagues detected significantly elevated alpha-synuclein mRNA levels in individual, surviving neuromelanin- and tyrosine hydroxylase-positive *substantia nigra* dopaminergic neurons from idiopathic PD brains compared to unaffected controls (Gründemann, J. et al. (2008) Elevated α-synuclein mRNA levels in individual UV-laser-microdissected dopaminergic substantia nigra neurons in idiopathic Parkinson's disease. NAR 36(7): e38). These data demonstrate a cell-type specific transcriptional upregulation of alpha-synuclein expression in neurons being a key population in PD.

It has also been found in *in-vitro* studies that methylation of human SNCA intron I decreased gene expression, while inhibition of DNA methylation activated SNCA expression. Notably, methylation of SNCA intron I was reduced in DNA from sporadic PD patient's substantia nigra putamen, and cortex (Jowaed, A. et al. (2010) Methylation regulates alpha-synuclein expression and is decreased in Parkinson's disease patients' brains. J. Neurosci. 30(18):6355-6359). However, methylation pattern in the SNCA region of interest varied considerably between tissues and individuals.

Besides of its association with Alzheimer's disease and Parkinson's disease, epigenetic regulation of the SNCA gene's expression appears to be involved in other disorders too. For example, an increased methylation of the alpha synuclein promoter DNA in patients with alcoholism was observed (Bönsch, D. et al. (2005) DNA hypermethylation of the alpha synuclein promoter in patients with alcoholism. Neuroreport. 8;16(2):167-170). The increased methylation of the alpha synuclein promoter DNA was significantly associated with elevated homocysteine levels in said patients. Since hypermethylation of DNA is an important epigenetic factor in down regulation of gene expression, and since alpha synuclein has been linked to craving, hypermethylation of alpha-synuclein promoter DNA was discussed to explain the reduced value of craving under alcohol drinking conditions. In contrast, alpha-synuclein protein expression was found to be significantly higher in patients with alcoholism when compared to healthy controls (Bönsch, D. et al. (2005) Alpha-synuclein protein levels are increased in alcoholic patients and are linked to craving. Alcohol Clin. Res. 29(5):763-765.).

Furthermore, global DNA hypomethylation and DNA hypermethylation of the alpha-synuclein promoter was observed in females with anorexia nervosa indicating epigenetic contribution to the pathophysiology of eating disorders. In patients with anorexia a decreased alpha-synuclein expression was associated with DNA hypermethylation of the alpha-synuclein promoter. (Frieling, H. et al. (2007) Global DNA hypomethylation and DNA hypermethylation of the alpha synuclein promoter in females with anorexia nervosa. Mol. Psychiatry 12:229-230).

The present invention is based on the surprising findings that a decreased methylation in the 5' region of the human SNCA gene, which was observed in DNA from cells of the central nervous system of patients having developed Parkinson's disease, can also be found in the DNA obtained from peripheral blood monocytes of patients having developed Parkinson's disease. These findings permit providing an inexpensive and easy to perform test for diagnosis of Parkinson's disease. Such a test is further advantageous, because it permits diagnosis of Parkinson's disease at a very early stage of the disease's progression and may even permit assessing a person's risk of developing Parkinson's disease when becoming older, even if no symptoms are present yet.

In a first aspect, the present invention concerns the use of peripheral blood monocytes of a patient for diagnosis of Parkinson's disease and/or assessing the patient's risk of developing Parkinson's disease, wherein the methylation pattern of the SNCA gene from DNA of said peripheral blood monocytes is determined.

In a further aspect, the present invention concerns a method of determining the methylation pattern of the SNCA gene from DNA of peripheral blood monocytes of a patient for diagnosis of Parkinson's disease and/or assessing the patient's risk of developing Parkinson's disease.

In yet another aspect, the present invention concerns a method for diagnosis and/or assessing a patient's risk of developing Parkinson's disease, wherein the methylation pattern of the SNCA gene from DNA of peripheral blood monocytes of said patient is determined.

The term "patient" refers to any person whose risk of developing Parkinson's disease during his or her lifetime is to be assessed or who is to be diagnosed for Parkinson's disease, regardless of whether clinical symptoms, in particular neurological symptoms being associated with Parkinson's disease, are present or not. Hence, the term "patient" is not restricted to persons already affected by Parkinson's disease, but also refers to healthy individuals.
Figure 1 displays the nucleotide sequence of a region (SNCA_{(-926/-483)}) within the first intron of the human SNCA gene and shows the predicted transcription factor binding sites adjacent to the CpG dinucleotides within SNCA_{(-926/-483)}.
Figure 2 shows the methylation pattern of the SNCA_{(-926/-483)} region of the human SNCA gene in peripheral blood monocytes of Parkinson patients (black columns) by displaying the percentage of methylated cysteine residues at each CpG dinucleotide in said region compared to neurologically healthy individuals (white columns).

According to the first aspect of the present invention, peripheral blood monocytes of a patient are used for diagnosis of whether the patient has developed Parkinson's disease and/or for assessing the patient's risk of developing Parkinson's disease, even if said patient does not shown any neurological symptoms associated with Parkinson's disease. For diagnosis of whether the patient has developed Parkinson's disease and/or for assessing the patient's risk of developing Parkinson's disease, in particular sporadic PD, the methylation pattern of the SNCA gene obtained from genomic DNA of the patient's peripheral blood monocytes is determined.

Using peripheral blood monocytes for diagnosis of whether the patient has developed Parkinson's disease and/or for assessing the patient's risk of developing Parkinson's disease is advantageous over methods currently used for diagnosing Parkinson's disease, because peripheral blood monocytes of a patient can be easily obtained without posing undue health hazards to the patient and without the need of expensive technical equipment. Moreover, peripheral blood monocytes can be used even if the patient does not suffer from neurological symptoms associated with Parkinson's disease. Therefore, the present invention provides an opportunity to determine whether a person is at risk of developing PD in the future. It may also be possible to use peripheral blood monocytes from a patient's blood sample that was taken for other reasons than the instant diagnosis or risk assessment. For example, using peripheral blood monocytes from blood samples of a patient that were taken at various points of time during the patient's life, alterations in the methylation pattern of the SNCA gene can be monitored without extensive costs such that the etiology of sporadically occurring PD may be determined.

In a preferred embodiment of the first aspect of the invention, the use of peripheral blood monocytes of a patient for diagnosing and/or assessing the patient's risk of developing Parkinson's disease is characterized in that the methylation pattern of intron 1 of the SNCA gene from genomic DNA of the patient's peripheral blood monocytes is determined.

In another preferred or additional embodiment of the first aspect of the invention, the use of peripheral blood monocytes of a patient for diagnosing and/or assessing the patient's risk of developing Parkinson's disease is characterized in that the methylation pattern of the SNCA_{(-926/-483)} region of the human SNCA gene from genomic DNA of the patient's peripheral blood monocytes is determined.

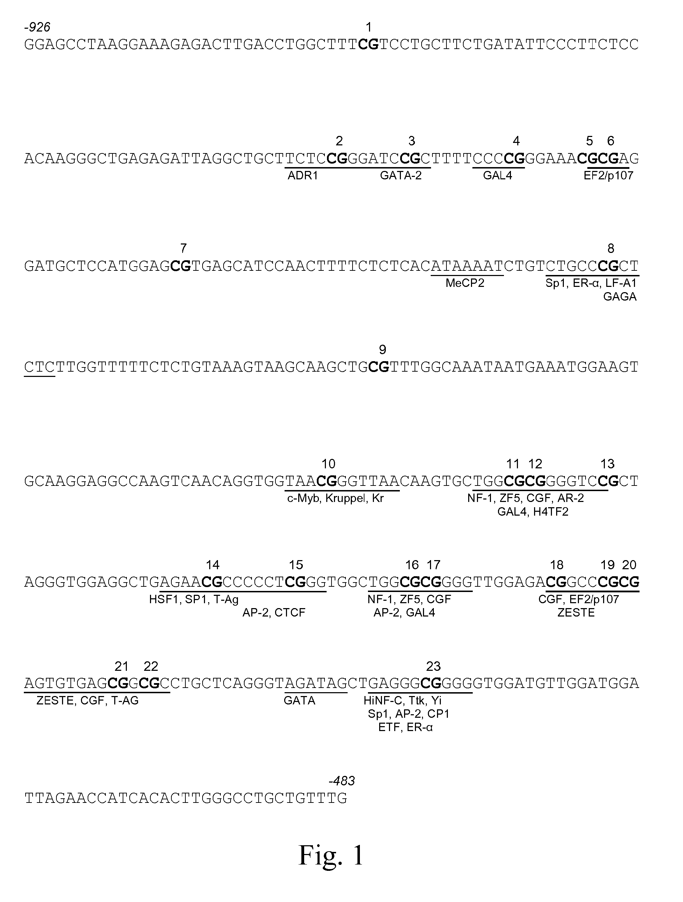

The SNCA_{(-926/-483)} region of the SNCA gene is the nucleotide sequence of the SNCA gene upstream of the ATG start codon for SNCA, consisting of the nucleotides sequence from nucleotide -926 to nucleotide -483 with respect to the ATG start codon of SNCA, the adenosine (A) of said start codon being designated nucleotide 1. Hence, the SNCA_{(-926/-483)} region is located upstream or 5' of the start codon as is identified by the minus in front of the designated position numbers. Notably, intron 1 of the human SNCA gene and hence the SNCA_{(-926/-483)} region of the human SNCA gene does not comprise the promoter of the human SNCA gene. The promoter of the human SNCA gene is located upstream, i. e. in 5' direction, of exon 1 which in turn is located upstream of and adjacent to said intron 1. The nucleotide sequence of SNCA_{(-926/-483)} region (SEQ ID NO: 1) of the human SNCA gene is shown in Figure 1.

The SNCA_{(-926/-483)} region of the human SNCA gene comprises 23 CpG dinucleotides, wherein the cytosine of each of these CpG dinucleotides may or may not be methylated. Whether the cytosine of a given CpG dinucleotide is methylated or not defines the methylation status of the cytosine of said particular CpG dinucleotide. The methylation pattern of a DNA molecule or a region of a DNA molecule, for example a gene such as the SNCA gene ,or of the SNCA_{(-926/-483)} region of the human SNCA gene, is defined by the combination of the methylation status of each CpG dinucleotide within said DNA molecule or said region. Table 1 provides an overview of the 23 CpG dinucleotides within the SNCA_{(-926/-483)} region of the human SNCA gene and their position within intron 1 with respect to the ATG start codon of the human SNCA gene.

**Table 1: List of the 23 CpG dinucleotides within the SNCA_{(-926/-483)} region of the human SNCA gene which may be subject to methylation. The position of each of these 23 CpG dinucleotides with reference to the ATG start codon of the human SNCA gene is provided, said start codon consisting of nucleotides 1 to 3 pursuant to the nomenclature used herein.**

| No. | Position | No. | Position | No. | Position |
|---|---|---|---|---|---|
| 1 | -894/-893 | 9 | -716/-715 | 17 | -593/-592 |
| 2 | -838/-837 | 10 | -662/-661 | 18 | -581/-580 |
| 3 | -831/-830 | 11 | -644/-643 | 19 | -576/-575 |
| 4 | -821/-820 | 12 | -642/-641 | 20 | -574/-573 |
| 5 | -814/-813 | 13 | -635/-634 | 21 | -564/-563 |
| 6 | -812/-811 | 14 | -614/-613 | 22 | -561/-560 |
| 7 | -794/-793 | 15 | -606/-605 | 23 | -534/-533 |
| 8 | -753/-752 | 16 | -595/-594 | | |

In another preferred or additional embodiment of the first aspect of the invention, the methylation status of one or more CpG dinucleotides of the SNCA_{(-926/-483)} region of the human SNCA gene is determined. Preferably said one or more CpG dinucleotides are selected from the group consisting of CpG dinucleotides No. 1, 2, 7, 8, 11, 12, 13, 18, 21, 22 and 23 of the SNCA_{(-926/-483)} region of the human SNCA gene. More preferably, said one or more CpG dinucleotides is/are selected from the group consisting of CpG dinucleotides No. 1, 7, 8, 13, 22 and 23 of the SNCA_{(-926/-483)} region of the human SNCA gene.

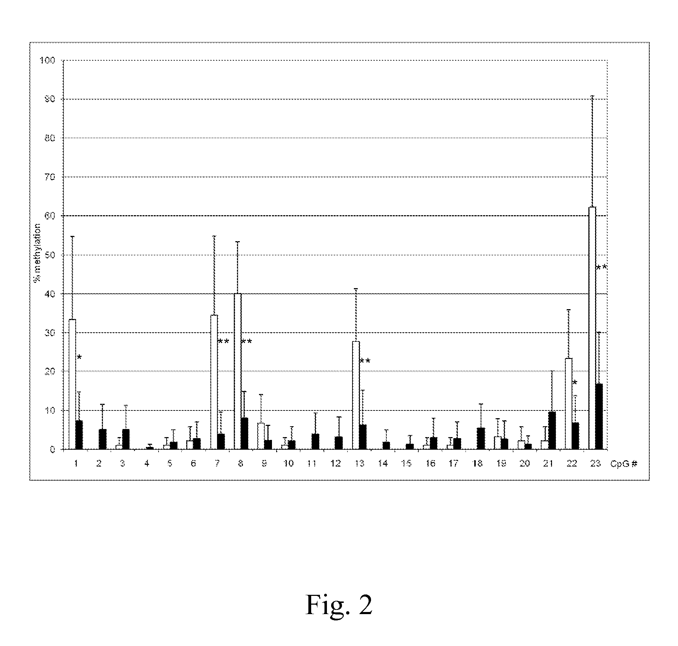

CpG dinucleotides No. 1, 7, 8, 13, 22 and 23 of the SNCA_{(-926/-483)} region of the human SNCA gene are preferred, because it was assessed that the methylation status of these particular CpG dinucleotides is significantly altered in patients suffering from Parkinson's disease, in particular from sporadic PD, compared to healthy control individuals. More specifically, the methylation pattern of the SNCA_{(-926/-483)} region of the human SNCA gene from peripheral blood monocytes as displayed in Figure 2 illustrates that the methylation status of CpG dinucleotides No. 1, 7, 8, 13, 22 and 23 is significantly altered in patients suffering from sporadic PD. In said patients, the percentage of methylated cytosines in at least one of CpG dinucleotides No. 1, 7, 8, 13, 22 and 23 is significantly reduced compared to the control DNA from healthy individuals. Hence, the SNCA_{(-926/-483)} region of the human SNCA gene from peripheral blood monocytes is hypomethylated in PD patients affected with sporadic PD. The SNCA_{(-926/-483)} region of the human SNCA gene from peripheral blood monocytes is hypomethylated with respect to its CpG dinucleotides No. 1, 7, 8, 13, 22 and 23.

In addition, the percentage of methylated CpG dinucleotide No. 2, 11, 12, 18 and 21 of the SNCA_{(-926/-483)} region of the human SNCA gene from peripheral blood monocytes appears to be increased in patients affected with sporadic PD. The evidence is striking although at present not statistically significant due to the low number of probes that were analyzed thus far. However, the methylation pattern of the SNCA_{(-926/-483)} region of the human SNCA gene from peripheral blood monocytes is altered in patients affected by PD in that said methylation pattern displays a hypermethylation with respect to CpG dinucleotide No. 2, 11, 12, 18 and 21 of the SNCA_{(-926/-483)} region of the human SNCA gene from peripheral blood monocytes.

Notwithstanding that virtually any method for analyzing the methylation status of a given CpG dinucleotide can be employed for analyzing the methylation status of the human SNCA gene, of intron 1 of the human SNCA gene, and/or of the SNCA_{(-926/-483)} region of the human SNCA gene, in particular of one or more CpG dinucleotides of the SNCA_{(-926/-483)} region of the human SNCA gene, preferably of one or more CpG dinculeotides of the SNCA_{(-926/-483)} region of the human SNCA gene selected from the group consisting of CpG dinucleotides No. 1, 2, 7, 8, 11, 12, 13, 18, 21, 22 and 23 of the SNCA_{(-926/-483)} region of the human SNCA gene, and more preferably of one or more CpG dinucleotides selected from the group consisting of CpG dinucleotides No. 1, 7, 8, 13, 22 and 23 of the SNCA_{(-926/-483)} region of the human SNCA gene, it is preferred that the methylation status is determined by one or more methods selected from the group consisting of methylation specific PCR, direct sequence analysis of bisulfite-treated DNA, COBRA-Assay and analysis of methylation-specific restriction enzyme digestion.

According to a preferred embodiment of the first aspect of the invention, the methylation pattern of the human SNCA-gene, of intron 1 of the human SNCA gene, or of the SNCA_{(-926/-483)} region of the human SNCA gene, in particular of one or more CpG dinucleotides of the SNCA_{(-926/-483)} region of the human SNCA gene, preferably of one or more CpG dinucleotides of the SNCA_{(-926/-483)} region of the human SNCA gene selected from the group consisting of CpG dinucleotides 1, 2, 7, 8, 11, 12, 13, 18, 21, 22 and 23 of the SNCA_{(-926/-483)} region of the human SNCA gene is determined by methylation specific PCR (MSP), and more preferably of one or more CpG dinucleotides selected from the group consisting of CpG dinucleotides No. 1, 7, 8, 13, 22 and 23 of the SNCA_{(-926/-483)} region of the human SNCA gene.

In animals DNA methylation predominantly involves the addition of a methyl group to the carbon-5 position of cytosine residues of the dinucleotide CpG, and is implicated in repression of transcriptional activity. Treatment of DNA with sodium bisulfite converts cytosine residues to uracil, but leaves 5-methylcytosine residues unaffected. Thus, sodium bisulfite treatment introduces specific changes in the DNA sequence that depend on the methylation status of individual cytosine residues, yielding single-nucleotide resolution information about the methylation status of a segment of DNA.

MSP can rapidly assess the methylation status of virtually any group of CpG dinucleotides within a CpG island, independent of the use of cloning or methylation-sensitive restriction enzymes. The MSP comprises initial modification of DNA by sodium bisulfite, converting all unmethylated, but not methylated, cytosines to uracil, and subsequent amplification with primers specific for methylated versus unmethylated DNA. MSP requires very small quantities of DNA, and is sensitive to 0.1 % methylated alleles of a given CpG island locus.

According to another preferred or additional embodiment of the first aspect of the invention, methylation of the human SNCA-gene, of intron 1 of the human SNCA gene, and/or of the SNCA_{(-926/-483)} region of the human SNCA gene, in particular of one or more CpG dinucleotides of the SNCA_{(-926/-483)} region of the human SNCA gene, preferably of one or more CpG dinculeotides of the SNCA_{(-926/-483)} region of the human SNCA gene selected from the group consisting of CpG dinucleotides 1, 2, 7, 8, 11, 12, 13, 18, 21, 22 and 23 of the SNCA_{(-926/-483)} region of the human SNCA gene and more preferably of one or more CpG dinucleotides selected from the group consisting of CpG dinucleotides No. 1, 7, 8, 13, 22 and 23 of the SNCA_{(-926/-483)} region of the human SNCA gene is determined by sequence analysis of bisulfite treated DNA.

The method of methylation analysis utilizing sequence analysis of bisulfite-treated DNA involves bisulfite treatment of the DNA to be analyzed, PCR amplification of the bisulfite treated DNA, cloning of the amplification product, and standard dideoxynucleotide DNA sequencing of the cloned DNA fragments to directly determine the nucleotides resistant to bisulfite conversion. Preferred primers for PCR amplification are designed to be strand-specific but not methylation-specific, i.e. the nucleotide sequence of the preferred primers do not comprise a sequence corresponding to a nucleotide sequence including a CpG dinucleotide. Hence, the preferred primers for PCR amplification flank but do not involve the methylation site or methylation sites of interest. However, in an additional or alternative embodiment, at least one or both, the forward primer and the reverse primer for PCR amplification may cover one or more CpG dinucleotides. To be strand specific and to allow amplification of methylated as well as non-methylated DNA fragments, a mixture of primers may be utilized, wherein the mixture of strand primers consists of primers having a pyrimidine nucleotide (Y) for the cytosine of the CpG dinucleotide within the DNA fragment to be amplified and covered by said strand primer, i. e. a C for amplification of said DNA fragment which is methylated at said C, or a T for amplification of said DNA fragment which is not methylated at said C. Therefore, the PCR amplification will amplify both methylated and unmethylated sequences, in contrast to methylation-specific PCR. All sites of unmethylated cytosines are displayed as thymines in the resulting amplified sequence of the sense strand, and as adenines in the amplified antisense strand. This method requires cloning of the PCR products prior to sequencing for adequate sensitivity. Alternatively, nested PCR methods can be used to enhance the product for sequencing.

Pyrosequencing may also be used to analyze bisulfite-treated DNA without using methylation-specific PCR. Following PCR amplification of the region of interest, pyrosequencing is used to determine the bisulfite-converted sequence of specific CpG sites in the region. The ratio of C-to-T at individual sites can be determined quantitatively based on the amount of C and T incorporation during the sequence extension.

According to another preferred or additional embodiment of the first aspect of the invention, methylation of the human SNCA-gene, of intron 1 of the human SNCA gene, or of the SNCA_{(-926/-483)} region of the human SNCA gene, in particular of one or more CpG dinucleotides of the SNCA_{(-926/-483)} region of the human SNCA gene, preferably of one or more CpG dinculeotides of the SNCA_{(-926/-483)} region of the human SNCA gene selected from the group consisting of CpG dinucleotides No. 1, 2, 7, 8, 11, 12, 13, 18, 21, 22 and 23 of the SNCA_{(-926/-483)} region of the human SNCA gene, and more preferably of one or more CpG dinucleotides selected from the group consisting of CpG dinucleotides No. 1, 7, 8, 13, 22 and 23 of the SNCA_{(-926/-483)} region of the human SNCA gene, is determined by combined bisulfite restriction analysis (COBRA assay).

The COBRA assay is a quantitative technique to determine DNA methylation levels at specific gene loci in small amounts of genomic DNA. In the COBRA assay, restriction enzyme digestion is used to reveal methylation dependent sequence differences in PCR products of sodium bisulfite-treated DNA. Methylation levels in the original DNA sample are represented by the relative amounts of digested and undigested PCR product in a linearly quantitative fashion across a wide spectrum of DNA methylation levels. The COBRA assay is easy to use and provides quantitative accuracy.

In the COBRA assay, methylation-dependent sequence differences are introduced into the genomic DNA by sodium bisulfite treatment and subsequent PCR amplification of the bisulfite treated DNA. This combination of bisulfite treatment and PCR amplification results in conversion of unmethylated cytosine residues to thymine and of methylated cytosine residues to cytosine. This sequence conversion can lead to methylation dependent creation of new restriction enzyme sites or it can lead to the methylation dependent retention of pre-existing restriction enzyme sites such as, for example, *Bs*tUI (CGCG). The primers used in the PCR amplification reaction do not contain CpG dinucleotides so that the amplification step does not discriminate between templates according to their original methylation status. Therefore, in the mixed population of DNA fragments resulting from said PCR, the fraction that has a newly created or retained restriction site that contains a CpG(s) directly reflects the percentage DNA methylation at that site in the original genomic DNA.

Aforementioned methods, i. e. MSP, direct sequencing of bisulfate-treated DNA, and COBRA, utilize oligonucleotides for analyzing the methylation pattern of a DNA fragment, for example as primers in PCR amplification reactions. For analyzing the methylation pattern of the human SNCA gene, of intron 1 of the human SNCA gene, or of the of the SNCA_{(-926/-483)} region of the human SNCA gene, preferably of one or more CpG dinucleotides of the SNCA_{(-926/-483)} region of the human SNCA gene selected from the group consisting of CpG dinucleotides No. 1, 2, 7, 8, 11, 12, 13, 18, 21, 22 and 23 of the SNCA_{(-926/-483)} region of the human SNCA gene, and more preferably of one or more CpG dinucleotides selected from the group consisting of CpG dinucleotides No. 1, 7, 8, 13, 22 and 23 of the SNCA_{(-926/-483)} region of the human SNCA gene, one or more oligonucleotides comprising a nucleotide sequence selected from the group consisting of SEQ ID NO: 5 to SEQ ID NO: 898 may be used.

Any one of said oligonucleotides comprising a nucleotide sequence selected from the group consisting of SEQ ID NO: 5 to SEQ ID NO: 898 may consist of 16, 17, 18, 19, 20, 21, 22 or more nucleotides. Preferred oligonucleotides for use in analyzing the methylation pattern of the human SNCA gene, of intron 1 of the human SNCA gene, or of the of the SNCA_{(-926/-483)} region of the human SNCA gene, preferably of one or more CpG dinculeotides of the SNCA_{(-926/-483)} region of the human SNCA gene selected from the group consisting of CpG dinucleotides No. 1, 2, 7, 8, 11, 12, 13, 18, 21, 22 and 23 of the SNCA_{(-926/-483)} region of the human SNCA gene, and more preferably of one or more CpG dinucleotides selected from the group consisting of CpG dinucleotides No. 1, 7, 8, 13, 22 and 23 of the SNCA_{(-926/-483)} region of the human SNCA gene, consist of 18 nucleotides having a nucleotide sequences selected from the group consisting of SEQ ID NO: 5 to SEQ ID NO: 898.

**Table 2: Preferred, sequence identical oligonucleotide sequences, specific for bisulfite converted methylated and/or non-methylated sense strand of the SNCA_{(-926/-483)} region of the human SNCA gene.**

| Sequence | SEQ ID NO: | Sequence | SEQ ID NO: |
|---|---|---|---|
| TTGGGGAGTTTAAGGAAA | 5 | AATAATGAAATGGAAGTG | 228 |
| TGGGGAGTTTAAGGAAAG | 6 | ATAATGAAATGGAAGTGT | 229 |
| GGGGAGTTTAAGGAAAGA | 7 | TAATGAAATGGAAGTGTA | 230 |
| GGGAGTTTAAGGAAAGAG | 8 | AATGAAATGGAAGTGTAA | 231 |
| GGAGTTTAAGGAAAGAGA | 9 | ATGAAATGGAAGTGTAAG | 232 |
| GAGTTTAAGGAAAGAGAT | 10 | TGAAATGGAAGTGTAAGG | 233 |
| AGTTTAAGGAAAGAGATT | 11 | GAAATGGAAGTGTAAGGA | 234 |
| GTTTAAGGAAAGAGATTT | 12 | AAATGGAAGTGTAAGGAG | 235 |
| TTTAAGGAAAGAGATTTG | 13 | AATGGAAGTGTAAGGAGG | 236 |
| TTAAGGAAAGAGATTTGA | 14 | ATGGAAGTGTAAGGAGGT | 237 |
| TAAGGAAAGAGATTTGAT | 15 | TGGAAGTGTAAGGAGGTT | 238 |
| AAGGAAAGAGATTTGATT | 16 | GGAAGTGTAAGGAGGTTA | 239 |
| AGGAAAGAGATTTGATTT | 17 | GAAGTGTAAGGAGGTTAA | 240 |
| GGAAAGAGATTTGATTTG | 18 | AAGTGTAAGGAGGTTAAG | 241 |
| GAAAGAGATTTGATTTGG | 19 | AGTGTAAGGAGGTTAAGT | 242 |
| AAAGAGATTTGATTTGGT | 20 | GTGTAAGGAGGTTAAGTT | 243 |
| AAGAGATTTGATTTGGTT | 21 | TGTAAGGAGGTTAAGTTA | 244 |
| AGAGATTTGATTTGGTTT | 22 | GTAAGGAGGTTAAGTTAA | 245 |
| GAGATTTGATTTGGTTTT | 23 | TAAGGAGGTTAAGTTAAT | 246 |
| AGATTTGATTTGGTTTTY | 24 | AAGGAGGTTAAGTTAATA | 247 |
| GATTTGATTTGGTTTTYG | 25 | AGGAGGTTAAGTTAATAG | 248 |
| ATTTGATTTGGTTTTYGT | 26 | GGAGGTTAAGTTAATAGG | 249 |
| TTTGATTTGGTTTTYGTT | 27 | GAGGTTAAGTTAATAGGT | 250 |
| TTGATTTGGTTTTYGTTT | 28 | AGGTTAAGTTAATAGGTG | 251 |
| TGATTTGGTTTTYGTTTT | 29 | GGTTAAGTTAATAGGTGG | 252 |
| GATTTGGTTTTYGTTTTG | 30 | GTTAAGTTAATAGGTGGT | 253 |
| ATTTGGTTTTYGTTTTGT | 31 | TTAAGTTAATAGGTGGTA | 254 |
| TTTGGTTTTYGTTTTGTT | 32 | TAAGTTAATAGGTGGTAA | 255 |
| TTGGTTTTYGTTTTGTTT | 33 | AAGTTAATAGGTGGTAAY | 256 |
| TGGTTTTYGTTTTGTTTT | 34 | AGTTAATAGGTGGTAAYG | 257 |
| GGTTTTYGTTTTGTTTTT | 35 | GTTAATAGGTGGTAAYGG | 258 |
| GTTTTYGTTTTGTTTTTG | 36 | TTAATAGGTGGTAAYGGG | 259 |
| TTTTYGTTTTGTTTTTGA | 37 | TAATAGGTGGTAAYGGGT | 260 |
| TTTYGTTTTGTTTTTGAT | 38 | AATAGGTGGTAAYGGGTT | 261 |
| TTYGTTTTGTTTTTGATA | 39 | ATAGGTGGTAAYGGGTTA | 262 |
| TYGTTTTGTTTTTGATAT | 40 | TAGGTGGTAAYGGGTTAA | 263 |
| YGTTTTGTTTTTGATATT | 41 | AGGTGGTAAYGGGTTAAT | 264 |
| GTTTTGTTTTTGATATTT | 42 | GGTGGTAAYGGGTTAATA | 265 |
| TTTTGTTTTTGATATTTT | 43 | GTGGTAAYGGGTTAATAA | 266 |
| TTTGTTTTTGATATTTTT | 44 | TGGTAAYGGGTTAATAAG | 267 |
| TTGTTTTTGATATTTTTT | 45 | GGTAAYGGGTTAATAAGT | 268 |
| TGTTTTTGATATTTTTTT | 46 | GTAAYGGGTTAATAAGTG | 269 |
| GTTTTTGATATTTTTTTT | 47 | TAAYGGGTTAATAAGTGT | 270 |
| TTTTTGATATTTTTTTTT | 48 | AAYGGGTTAATAAGTGTT | 271 |
| TTTTGATATTTTTTTTTT | 49 | AYGGGTTAATAAGTGTTG | 272 |
| TTTGATATTTTTTTTTTT | 50 | YGGGTTAATAAGTGTTGG | 273 |
| TTGATATTTTTTTTTTTA | 51 | GGGTTAATAAGTGTTGGY | 274 |
| TGATATTTTTTTTTTTAT | 52 | GGTTAATAAGTGTTGGYG | 275 |
| GATATTTTTTTTTTTATA | 53 | GTTAATAAGTGTTGGYGY | 276 |
| ATATTTTTTTTTTTATAA | 54 | TTAATAAGTGTTGGYGYG | 277 |
| TATTTTTTTTTTTATAAG | 55 | TAATAAGTGTTGGYGYGG | 278 |
| ATTTTTTTTTTTATAAGG | 56 | AATAAGTGTTGGYGYGGG | 279 |
| TTTTTTTTTTTATAAGGG | 57 | ATAAGTGTTGGYGYGGGG | 280 |
| TTTTTTTTTTATAAGGGT | 58 | TAAGTGTTGGYGYGGGGT | 281 |
| TTTTTTTTTATAAGGGTT | 59 | AAGTGTTGGYGYGGGGTT | 282 |
| TTTTTTTTATAAGGGTTG | 60 | AGTGTTGGYGYGGGGTTY | 283 |
| TTTTTTTATAAGGGTTGA | 61 | GTGTTGGYGYGGGGTTYG | 284 |
| TTTTTTATAAGGGTTGAG | 62 | TGTTGGYGYGGGGTTYGT | 285 |
| TTTTTATAAGGGTTGAGA | 63 | GTTGGYGYGGGGTTYGTT | 286 |
| TTTTATAAGGGTTGAGAG | 64 | TTGGYGYGGGGTTYGTTA | 287 |
| TTTATAAGGGTTGAGAGA | 65 | TGGYGYGGGGTTYGTTAG | 288 |
| TTATAAGGGTTGAGAGAT | 66 | GGYGYGGGGTTYGTTAGG | 289 |
| TATAAGGGTTGAGAGATT | 67 | GYGYGGGGTTYGTTAGGG | 290 |
| ATAAGGGTTGAGAGATTA | 68 | YGYGGGGTTYGTTAGGGT | 291 |
| TAAGGGTTGAGAGATTAG | 69 | GYGGGGTTYGTTAGGGTG | 292 |
| AAGGGTTGAGAGATTAGG | 70 | YGGGGTTYGTTAGGGTGG | 293 |
| AGGGTTGAGAGATTAGGT | 71 | GGGGTTYGTTAGGGTGGA | 294 |
| GGGTTGAGAGATTAGGTT | 72 | GGGTTYGTTAGGGTGGAG | 295 |
| GGTTGAGAGATTAGGTTG | 73 | GGTTYGTTAGGGTGGAGG | 296 |
| GTTGAGAGATTAGGTTGT | 74 | GTTYGTTAGGGTGGAGGT | 297 |
| TTGAGAGATTAGGTTGTT | 75 | TTYGTTAGGGTGGAGGTT | 298 |
| TGAGAGATTAGGTTGTTT | 76 | TYGTTAGGGTGGAGGTTG | 299 |
| GAGAGATTAGGTTGTTTT | 77 | TYGTTAGGGTGGAGGTTG | 300 |
| AGAGATTAGGTTGTTTTT | 78 | YGTTAGGGTGGAGGTTGA | 301 |
| GAGATTAGGTTGTTTTTT | 79 | GTTAGGGTGGAGGTTGAG | 302 |
| AGATTAGGTTGTTTTTTY | 80 | TTAGGGTGGAGGTTGAGA | 303 |
| GATTAGGTTGTTTTTTYG | 81 | TAGGGTGGAGGTTGAGAA | 304 |
| ATTAGGTTGTTTTTTYGG | 82 | AGGGTGGAGGTTGAGAAY | 305 |
| TTAGGTTGTTTTTTYGGG | 83 | GGGTGGAGGTTGAGAAYG | 306 |
| TAGGTTGTTTTTTYGGGA | 84 | GGTGGAGGTTGAGAAYGT | 307 |
| AGGTTGTTTTTTYGGGAT | 85 | GTGGAGGTTGAGAAYGTT | 308 |
| GGTTGTTTTTTYGGGATT | 86 | TGGAGGTTGAGAAYGTTT | 309 |
| GTTGTTTTTTYGGGATTY | 87 | GGAGGTTGAGAAYGTTTT | 310 |
| TTGTTTTTTYGGGATTYG | 88 | GAGGTTGAGAAYGTTTTT | 311 |
| TGTTTTTTYGGGATTYGT | 89 | AGGTTGAGAAYGTTTTTT | 312 |
| GTTTTTTYGGGATTYGTT | 90 | GGTTGAGAAYGTTTTTTY | 313 |
| TTTTTTYGGGATTYGTTT | 91 | GTTGAGAAYGTTTTTTYG | 314 |
| TTTTTYGGGATTYGTTTT | 92 | TTGAGAAYGTTTTTTYGG | 315 |
| TTTTYGGGATTYGTTTTT | 93 | TGAGAAYGTTTTTTYGGG | 316 |
| TTTYGGGATTYGTTTTTT | 94 | GAGAAYGTTTTTTYGGGT | 317 |
| TTYGGGATTYGTTTTTTT | 95 | AGAAYGTTTTTTYGGGTG | 318 |
| TYGGGATTYGTTTTTTTT | 96 | GAAYGTTTTTTYGGGTGG | 319 |
| YGGGATTYGTTTTTTTTY | 97 | AAYGTTTTTTYGGGTGGT | 320 |
| GGGATTYGTTTTTTTTYG | 98 | AYGTTTTTTYGGGTGGTT | 321 |
| GGATTYGTTTTTTTTYGG | 99 | YGTTTTTTYGGGTGGTTG | 322 |
| GATTYGTTTTTTTTYGGG | 100 | GTTTTTTYGGGTGGTTGG | 323 |
| ATTYGTTTTTTTTYGGGA | 101 | TTTTTTYGGGTGGTTGGY | 324 |
| TTYGTTTTTTTTYGGGAA | 102 | TTTTTYGGGTGGTTGGYG | 325 |
| TYGTTTTTTTTYGGGAAA | 103 | TTTTYGGGTGGTTGGYGY | 326 |
| YGTTTTTTTTYGGGAAAY | 104 | TTTYGGGTGGTTGGYGYG | 327 |
| GTTTTTTTTYGGGAAAYG | 105 | TTYGGGTGGTTGGYGYGG | 328 |
| TTTTTTTTYGGGAAAYGY | 106 | TYGGGTGGTTGGYGYGGG | 329 |
| TTTTTTTYGGGAAAYGYG | 107 | YGGGTGGTTGGYGYGGGG | 330 |
| TTTTTTYGGGAAAYGYGA | 108 | GGGTGGTTGGYGYGGGGT | 331 |
| TTTTTYGGGAAAYGYGAG | 109 | GGTGGTTGGYGYGGGGTT | 332 |
| TTTTYGGGAAAYGYGAGG | 110 | GTGGTTGGYGYGGGGTTG | 333 |
| TTTYGGGAAAYGYGAGGA | 111 | TGGTTGGYGYGGGGTTGG | 334 |
| TTYGGGAAAYGYGAGGAT | 112 | GGTTGGYGYGGGGTTGGA | 335 |
| TYGGGAAAYGYGAGGATG | 113 | GTTGGYGYGGGGTTGGAG | 336 |
| YGGGAAAYGYGAGGATGT | 114 | TTGGYGYGGGGTTGGAGA | 337 |
| GGGAAAYGYGAGGATGTT | 115 | TGGYGYGGGGTTGGAGAY | 338 |
| GGAAAYGYGAGGATGTTT | 116 | GGYGYGGGGTTGGAGAYG | 339 |
| GAAAYGYGAGGATGTTTT | 117 | GYGYGGGGTTGGAGAYGG | 340 |
| AAAYGYGAGGATGTTTTA | 118 | YGYGGGGTTGGAGAYGGT | 341 |
| AAYGYGAGGATGTTTTAT | 119 | GYGGGGTTGGAGAYGGTT | 342 |
| AYGYGAGGATGTTTTATG | 120 | YGGGGTTGGAGAYGGTTY | 343 |
| YGYGAGGATGTTTTATGG | 121 | GGGGTTGGAGAYGGTTYG | 344 |
| GYGAGGATGTTTTATGGA | 122 | GGGTTGGAGAYGGTTYGY | 345 |
| YGAGGATGTTTTATGGAG | 123 | GGTTGGAGAYGGTTYGYG | 346 |
| GAGGATGTTTTATGGAGY | 124 | GTTGGAGAYGGTTYGYGA | 347 |
| AGGATGTTTTATGGAGYG | 125 | TTGGAGAYGGTTYGYGAG | 348 |
| GGATGTTTTATGGAGYGT | 126 | TGGAGAYGGTTYGYGAGT | 349 |
| GATGTTTTATGGAGYGTG | 127 | GGAGAYGGTTYGYGAGTG | 350 |
| ATGTTTTATGGAGYGTGA | 128 | GAGAYGGTTYGYGAGTGT | 351 |
| TGTTTTATGGAGYGTGAG | 129 | AGAYGGTTYGYGAGTGTG | 352 |
| GTTTTATGGAGYGTGAGT | 130 | GAYGGTTYGYGAGTGTGA | 353 |
| TTTTATGGAGYGTGAGTA | 131 | AYGGTTYGYGAGTGTGAG | 354 |
| TTTATGGAGYGTGAGTAT | 132 | YGGTTYGYGAGTGTGAGY | 355 |
| TTATGGAGYGTGAGTATT | 133 | GGTTYGYGAGTGTGAGYG | 356 |
| TATGGAGYGTGAGTATTT | 134 | GTTYGYGAGTGTGAGYGG | 357 |
| ATGGAGYGTGAGTATTTA | 135 | TTYGYGAGTGTGAGYGGY | 358 |
| TGGAGYGTGAGTATTTAA | 136 | TYGYGAGTGTGAGYGGYG | 359 |
| GGAGYGTGAGTATTTAAT | 137 | YGYGAGTGTGAGYGGYGT | 360 |
| GAGYGTGAGTATTTAATT | 138 | GYGAGTGTGAGYGGYGTT | 361 |
| AGYGTGAGTATTTAATTT | 139 | YGAGTGTGAGYGGYGTTT | 362 |
| GYGTGAGTATTTAATTTT | 140 | GAGTGTGAGYGGYGTTTG | 363 |
| YGTGAGTATTTAATTTTT | 141 | AGTGTGAGYGGYGTTTGT | 364 |
| GTGAGTATTTAATTTTTT | 142 | GTGTGAGYGGYGTTTGTT | 365 |
| TGAGTATTTAATTTTTTT | 143 | TGTGAGYGGYGTTTGTTT | 366 |
| GAGTATTTAATTTTTTTT | 144 | GTGAGYGGYGTTTGTTTA | 367 |
| AGTATTTAATTTTTTTTT | 145 | TGAGYGGYGTTTGTTTAG | 368 |
| GTATTTAATTTTTTTTTT | 146 | GAGYGGYGTTTGTTTAGG | 369 |
| TATTTAATTTTTTTTTTA | 147 | AGYGGYGTTTGTTTAGGG | 370 |
| ATTTAATTTTTTTTTTAT | 148 | GYGGYGTTTGTTTAGGGT | 371 |
| TTTAATTTTTTTTTTATA | 149 | YGGYGTTTGTTTAGGGTA | 372 |
| TTAATTTTTTTTTTATAT | 150 | GGYGTTTGTTTAGGGTAG | 373 |
| TAATTTTTTTTTTATATA | 151 | GYGTTTGTTTAGGGTAGA | 374 |
| AATTTTTTTTTTATATAA | 152 | YGTTTGTTTAGGGTAGAT | 375 |
| ATTTTTTTTTTATATAAA | 153 | GTTTGTTTAGGGTAGATA | 376 |
| TTTTTTTTTTATATAAAA | 154 | TTTGTTTAGGGTAGATAG | 377 |
| TTTTTTTTTATATAAAAT | 155 | TTGTTTAGGGTAGATAGT | 378 |
| TTTTTTTTATATAAAATT | 156 | TGTTTAGGGTAGATAGTT | 379 |
| TTTTTTTATATAAAATTT | 157 | GTTTAGGGTAGATAGTTG | 380 |
| TTTTTTATATAAAATTTG | 158 | TTTAGGGTAGATAGTTGA | 381 |
| TTTTTATATAAAATTTGT | 159 | TTAGGGTAGATAGTTGAG | 382 |
| TTTTATATAAAATTTGTT | 160 | TAGGGTAGATAGTTGAGG | 383 |
| TTTATATAAAATTTGTTT | 161 | AGGGTAGATAGTTGAGGG | 384 |
| TTATATAAAATTTGTTTG | 162 | GGGTAGATAGTTGAGGGY | 385 |
| TATATAAAATTTGTTTGT | 163 | GGTAGATAGTTGAGGGYG | 386 |
| ATATAAAATTTGTTTGTT | 164 | GTAGATAGTTGAGGGYGG | 387 |
| TATAAAATTTGTTTGTTY | 165 | TAGATAGTTGAGGGYGGG | 388 |
| ATAAAATTTGTTTGTTYG | 166 | AGATAGTTGAGGGYGGGG | 389 |
| TAAAATTTGTTTGTTYGT | 167 | GATAGTTGAGGGYGGGGG | 390 |
| AAAATTTGTTTGTTYGTT | 168 | ATAGTTGAGGGYGGGGGT | 391 |
| AAATTTGTTTGTTYGTTT | 169 | TAGTTGAGGGYGGGGGTG | 392 |
| AATTTGTTTGTTYGTTTT | 170 | AGTTGAGGGYGGGGGTGG | 393 |
| ATTTGTTTGTTYGTTTTT | 171 | GTTGAGGGYGGGGGTGGA | 394 |
| TTTGTTTGTTYGTTTTTT | 172 | TTGAGGGYGGGGGTGGAT | 395 |
| TTGTTTGTTYGTTTTTTT | 173 | TGAGGGYGGGGGTGGATG | 396 |
| TGTTTGTTYGTTTTTTTG | 174 | GAGGGYGGGGGTGGATGT | 397 |
| GTTTGTTYGTTTTTTTGG | 175 | AGGGYGGGGGTGGATGTT | 398 |
| TTTGTTYGTTTTTTTGGT | 176 | GGGYGGGGGTGGATGTTG | 399 |
| TTGTTYGTTTTTTTGGTT | 177 | GGYGGGGGTGGATGTTGG | 400 |
| TGTTYGTTTTTTTGGTTT | 178 | GYGGGGGTGGATGTTGGA | 401 |
| GTTYGTTTTTTTGGTTTT | 179 | YGGGGGTGGATGTTGGAT | 402 |
| TTYGTTTTTTTGGTTTTT | 180 | GGGGGTGGATGTTGGATG | 403 |
| TYGTTTTTTTGGTTTTTT | 181 | GGGGTGGATGTTGGATGG | 404 |
| YGTTTTTTTGGTTTTTTT | 182 | GGGTGGATGTTGGATGGA | 405 |
| GTTTTTTTGGTTTTTTTT | 183 | GGTGGATGTTGGATGGAT | 406 |
| TTTTTTTGGTTTTTTTTT | 184 | GTGGATGTTGGATGGATT | 407 |
| TTTTTTGGTTTTTTTTTG | 185 | TGGATGTTGGATGGATTA | 408 |
| TTTTTGGTTTTTTTTTGT | 186 | GGATGTTGGATGGATTAG | 409 |
| TTTTGGTTTTTTTTTGTA | 187 | GATGTTGGATGGATTAGA | 410 |
| TTTGGTTTTTTTTTGTAA | 188 | ATGTTGGATGGATTAGAA | 411 |
| TTGGTTTTTTTTTGTAAA | 189 | TGTTGGATGGATTAGAAT | 412 |
| TGGTTTTTTTTTGTAAAG | 190 | GTTGGATGGATTAGAATT | 413 |
| GGTTTTTTTTTGTAAAGT | 191 | TTGGATGGATTAGAATTA | 414 |
| GTTTTTTTTTGTAAAGTA | 192 | TGGATGGATTAGAATTAT | 415 |
| TTTTTTTTTGTAAAGTAA | 193 | GGATGGATTAGAATTATT | 416 |
| TTTTTTTTGTAAAGTAAG | 194 | GATGGATTAGAATTATTA | 417 |
| TTTTTTTGTAAAGTAAGT | 195 | ATGGATTAGAATTATTAT | 418 |
| TTTTTTGTAAAGTAAGTA | 196 | TGGATTAGAATTATTATA | 419 |
| TTTTTGTAAAGTAAGTAA | 197 | GGATTAGAATTATTATAT | 420 |
| TTTTGTAAAGTAAGTAAG | 198 | GATTAGAATTATTATATT | 421 |
| TTTGTAAAGTAAGTAAGT | 199 | ATTAGAATTATTATATTT | 422 |
| TTGTAAAGTAAGTAAGTT | 200 | TTAGAATTATTATATTTG | 423 |
| TGTAAAGTAAGTAAGTTG | 201 | TAGAATTATTATATTTGG | 424 |
| GTAAAGTAAGTAAGTTGY | 202 | AGAATTATTATATTTGGG | 425 |
| TAAAGTAAGTAAGTTGYG | 203 | GAATTATTATATTTGGGT | 426 |
| AAAGTAAGTAAGTTGYGT | 204 | AATTATTATATTTGGGTT | 427 |
| AAGTAAGTAAGTTGYGTT | 205 | ATTATTATATTTGGGTTT | 428 |
| AGTAAGTAAGTTGYGTTT | 206 | TTATTATATTTGGGTTTG | 429 |
| GTAAGTAAGTTGYGTTTG | 207 | TATTATATTTGGGTTTGT | 430 |
| TAAGTAAGTTGYGTTTGG | 208 | ATTATATTTGGGTTTGTT | 431 |
| AAGTAAGTTGYGTTTGGT | 209 | TTATATTTGGGTTTGTTG | 432 |
| AGTAAGTTGYGTTTGGTA | 210 | TATATTTGGGTTTGTTGT | 433 |
| GTAAGTTGYGTTTGGTAA | 211 | ATATTTGGGTTTGTTGTT | 434 |
| TAAGTTGYGTTTGGTAAA | 212 | TATTTGGGTTTGTTGTTT | 435 |
| AAGTTGYGTTTGGTAAAT | 213 | ATTTGGGTTTGTTGTTTG | 436 |
| AGTTGYGTTTGGTAAATA | 214 | TTTGGGTTTGTTGTTTGT | 437 |
| GTTGYGTTTGGTAAATAA | 215 | TTGGGTTTGTTGTTTGTT | 438 |
| TTGYGTTTGGTAAATAAT | 216 | TGGGTTTGTTGTTTGTTT | 439 |
| TGYGTTTGGTAAATAATG | 217 | GGGTTTGTTGTTTGTTTG | 440 |
| GYGTTTGGTAAATAATGA | 218 | GGTTTGTTGTTTGTTTGA | 441 |
| YGTTTGGTAAATAATGAA | 219 | GTTTGTTGTTTGTTTGAG | 442 |
| GTTTGGTAAATAATGAAA | 220 | TTTGTTGTTTGTTTGAGT | 443 |
| TTTGGTAAATAATGAAAT | 221 | TTGTTGTTTGTTTGAGTT | 444 |
| TTGGTAAATAATGAAATG | 222 | TGTTGTTTGTTTGAGTTT | 445 |
| TGGTAAATAATGAAATGG | 223 | GTTGTTTGTTTGAGTTTG | 446 |
| GGTAAATAATGAAATGGA | 224 | TTGTTTGTTTGAGTTTGA | 447 |
| GTAAATAATGAAATGGAA | 225 | TGTTTGTTTGAGTTTGAA | 448 |
| TAAATAATGAAATGGAAG | 226 | GTTTGTTTGAGTTTGAAT | 449 |
| AAATAATGAAATGGAAGT | 227 | TTTGTTTGAGTTTGAATT | 450 |
| | | TTGTTTGAGTTTGAATTA | 451 |

**Table 3: Preferred, complementary oligonucleotide sequences, specific for bisulfite converted methylated and/or non-methylated antisense strand of the SNCA_{(-926/-483)} region of the human SNCA gene.**

| Sequence | SEQ ID NO: | Sequence | SEQ ID NO: |
|---|---|---|---|
| TAATTCAAACTCAAACAA | 452 | ACTTCCATTTCATTATTT | 675 |
| AATTCAAACTCAAACAAA | 453 | CTTCCATTTCATTATTTA | 676 |
| ATTCAAACTCAAACAAAC | 454 | TTCCATTTCATTATTTAC | 677 |
| TTCAAACTCAAACAAACA | 455 | TCCATTTCATTATTTACC | 678 |
| TCAAACTCAAACAAACAA | 456 | CCATTTCATTATTTACCA | 679 |
| CAAACTCAAACAAACAAC | 457 | CATTTCATTATTTACCAA | 680 |
| AAACTCAAACAAACAACA | 458 | ATTTCATTATTTACCAAA | 681 |
| AACTCAAACAAACAACAA | 459 | TTTCATTATTTACCAAAC | 682 |
| ACTCAAACAAACAACAAA | 460 | TTCATTATTTACCAAACR | 683 |
| CTCAAACAAACAACAAAC | 461 | TCATTATTTACCAAACRC | 684 |
| TCAAACAAACAACAAACC | 462 | CATTATTTACCAAACRCA | 685 |
| CAAACAAACAACAAACCC | 463 | ATTATTTACCAAACRCAA | 686 |
| AAACAAACAACAAACCCA | 464 | TTATTTACCAAACRCAAC | 687 |
| AACAAACAACAAACCCAA | 465 | TATTTACCAAACRCAACT | 688 |
| ACAAACAACAAACCCAAA | 466 | ATTTACCAAACRCAACTT | 689 |
| CAAACAACAAACCCAAAT | 467 | TTTACCAAACRCAACTTA | 690 |
| AAACAACAAACCCAAATA | 468 | TTACCAAACRCAACTTAC | 691 |
| AACAACAAACCCAAATAT | 469 | TACCAAACRCAACTTACT | 692 |
| ACAACAAACCCAAATATA | 470 | ACCAAACRCAACTTACTT | 693 |
| CAACAAACCCAAATATAA | 471 | CCAAACRCAACTTACTTA | 694 |
| AACAAACCCAAATATAAT | 472 | CAAACRCAACTTACTTAC | 695 |
| ACAAACCCAAATATAATA | 473 | AAACRCAACTTACTTACT | 696 |
| CAAACCCAAATATAATAA | 474 | AACRCAACTTACTTACTT | 697 |
| AAACCCAAATATAATAAT | 475 | ACRCAACTTACTTACTTT | 698 |
| AACCCAAATATAATAATT | 476 | CRCAACTTACTTACTTTA | 699 |
| ACCCAAATATAATAATTC | 477 | RCAACTTACTTACTTTAC | 700 |
| CCCAAATATAATAATTCT | 478 | CAACTTACTTACTTTACA | 701 |
| CCAAATATAATAATTCTA | 479 | AACTTACTTACTTTACAA | 702 |
| CAAATATAATAATTCTAA | 480 | ACTTACTTACTTTACAAA | 703 |
| AAATATAATAATTCTAAT | 481 | CTTACTTACTTTACAAAA | 704 |
| AATATAATAATTCTAATC | 482 | TTACTTACTTTACAAAAA | 705 |
| ATATAATAATTCTAATCC | 483 | TACTTACTTTACAAAAAA | 706 |
| TATAATAATTCTAATCCA | 484 | ACTTACTTTACAAAAAAA | 707 |
| ATAATAATTCTAATCCAT | 485 | CTTACTTTACAAAAAAAA | 708 |
| TAATAATTCTAATCCATC | 486 | TTACTTTACAAAAAAAAA | 709 |
| AATAATTCTAATCCATCC | 487 | TACTTTACAAAAAAAAAC | 710 |
| ATAATTCTAATCCATCCA | 488 | ACTTTACAAAAAAAAACC | 711 |
| TAATTCTAATCCATCCAA | 489 | CTTTACAAAAAAAAACCA | 712 |
| AATTCTAATCCATCCAAC | 490 | TTTACAAAAAAAAACCAA | 713 |
| ATTCTAATCCATCCAACA | 491 | TTACAAAAAAAAACCAAA | 714 |
| TTCTAATCCATCCAACAT | 492 | TACAAAAAAAAACCAAAA | 715 |
| TCTAATCCATCCAACATC | 493 | ACAAAAAAAAACCAAAAA | 716 |
| CTAATCCATCCAACATCC | 494 | CAAAAAAAAACCAAAAAA | 717 |
| TAATCCATCCAACATCCA | 495 | AAAAAAAAACCAAAAAAA | 718 |
| AATCCATCCAACATCCAC | 496 | AAAAAAAACCAAAAAAAC | 719 |
| ATCCATCCAACATCCACC | 497 | AAAAAAACCAAAAAAACR | 720 |
| TCCATCCAACATCCACCC | 498 | AAAAAACCAAAAAAACRA | 721 |
| CCATCCAACATCCACCCC | 499 | AAAAACCAAAAAAACRAA | 722 |
| CATCCAACATCCACCCCC | 500 | AAAACCAAAAAAACRAAC | 723 |
| ATCCAACATCCACCCCCR | 501 | AAACCAAAAAAACRAACA | 724 |
| TCCAACATCCACCCCCRC | 502 | AACCAAAAAAACRAACAA | 725 |
| CCAACATCCACCCCCRCC | 503 | ACCAAAAAAACRAACAAA | 726 |
| CAACATCCACCCCCRCCC | 504 | CCAAAAAAACRAACAAAC | 727 |
| AACATCCACCCCCRCCCT | 505 | CAAAAAAACRAACAAACA | 728 |
| ACATCCACCCCCRCCCTC | 506 | AAAAAAACRAACAAACAA | 729 |
| CATCCACCCCCRCCCTCA | 507 | AAAAAACRAACAAACAAA | 730 |
| ATCCACCCCCRCCCTCAA | 508 | AAAAACRAACAAACAAAT | 731 |
| TCCACCCCCRCCCTCAAC | 509 | AAAACRAACAAACAAATT | 732 |
| CCACCCCCRCCCTCAACT | 510 | AAACRAACAAACAAATTT | 733 |
| CACCCCCRCCCTCAACTA | 511 | AACRAACAAACAAATTTT | 734 |
| ACCCCCRCCCTCAACTAT | 512 | ACRAACAAACAAATTTTA | 735 |
| CCCCCRCCCTCAACTATC | 513 | CRAACAAACAAATTTTAT | 736 |
| CCCCRCCCTCAACTATCT | 514 | RAACAAACAAATTTTATA | 737 |
| CCCRCCCTCAACTATCTA | 515 | AACAAACAAATTTTATAT | 738 |
| CCRCCCTCAACTATCTAC | 516 | ACAAACAAATTTTATATA | 739 |
| CRCCCTCAACTATCTACC | 517 | CAAACAAATTTTATATAA | 740 |
| RCCCTCAACTATCTACCC | 518 | AAACAAATTTTATATAAA | 741 |
| CCCTCAACTATCTACCCT | 519 | AACAAATTTTATATAAAA | 742 |
| CCTCAACTATCTACCCTA | 520 | ACAAATTTTATATAAAAA | 743 |
| CTCAACTATCTACCCTAA | 521 | CAAATTTTATATAAAAAA | 744 |
| TCAACTATCTACCCTAAA | 522 | AAATTTTATATAAAAAAA | 745 |
| CAACTATCTACCCTAAAC | 523 | AATTTTATATAAAAAAAA | 746 |
| AACTATCTACCCTAAACA | 524 | ATTTTATATAAAAAAAAA | 747 |
| ACTATCTACCCTAAACAA | 525 | TTTTATATAAAAAAAAAA | 748 |
| CTATCTACCCTAAACAAA | 526 | TTTATATAAAAAAAAAAT | 749 |
| TATCTACCCTAAACAAAC | 527 | TTATATAAAAAAAAAATT | 750 |
| ATCTACCCTAAACAAACR | 528 | TATATAAAAAAAAAATTA | 751 |
| TCTACCCTAAACAAACRC | 529 | ATATAAAAAAAAAATTAA | 752 |
| CTACCCTAAACAAACRCC | 530 | TATAAAAAAAAAATTAAA | 753 |
| TACCCTAAACAAACRCCR | 531 | ATAAAAAAAAAATTAAAT | 754 |
| ACCCTAAACAAACRCCRC | 532 | TAAAAAAAAAATTAAATA | 755 |
| CCCTAAACAAACRCCRCT | 533 | AAAAAAAAAATTAAATAC | 756 |
| CCTAAACAAACRCCRCTC | 534 | AAAAAAAAATTAAATACT | 757 |
| CTAAACAAACRCCRCTCA | 535 | AAAAAAAATTAAATACTC | 758 |
| TAAACAAACRCCRCTCAC | 536 | AAAAAAATTAAATACTCA | 759 |
| AAACAAACRCCRCTCACA | 537 | AAAAAATTAAATACTCAC | 760 |
| AACAAACRCCRCTCACAC | 538 | AAAAATTAAATACTCACR | 761 |
| ACAAACRCCRCTCACACT | 539 | AAAATTAAATACTCACRC | 762 |
| CAAACRCCRCTCACACTC | 540 | AAATTAAATACTCACRCT | 763 |
| AAACRCCRCTCACACTCR | 541 | AATTAAATACTCACRCTC | 764 |
| AACRCCRCTCACACTCRC | 542 | ATTAAATACTCACRCTCC | 765 |
| ACRCCRCTCACACTCRCR | 543 | TTAAATACTCACRCTCCA | 766 |
| CRCCRCTCACACTCRCRA | 544 | TAAATACTCACRCTCCAT | 767 |
| RCCRCTCACACTCRCRAA | 545 | TAAATACTCACRCTCCAT | 768 |
| CCRCTCACACTCRCRAAC | 546 | AAATACTCACRCTCCATA | 769 |
| CRCTCACACTCRCRAACC | 547 | AATACTCACRCTCCATAA | 770 |
| RCTCACACTCRCRAACCR | 548 | ATACTCACRCTCCATAAA | 771 |
| CTCACACTCRCRAACCRT | 549 | TACTCACRCTCCATAAAA | 772 |
| TCACACTCRCRAACCRTC | 550 | ACTCACRCTCCATAAAAC | 773 |
| CACACTCRCRAACCRTCT | 551 | CTCACRCTCCATAAAACA | 774 |
| ACACTCRCRAACCRTCTC | 552 | TCACRCTCCATAAAACAT | 775 |
| CACTCRCRAACCRTCTCC | 553 | CACRCTCCATAAAACATC | 776 |
| ACTCRCRAACCRTCTCCA | 554 | ACRCTCCATAAAACATCC | 777 |
| CTCRCRAACCRTCTCCAA | 555 | CRCTCCATAAAACATCCT | 778 |
| TCRCRAACCRTCTCCAAC | 556 | RCTCCATAAAACATCCTC | 779 |
| CRCRAACCRTCTCCAACC | 557 | CTCCATAAAACATCCTCR | 780 |
| RCRAACCRTCTCCAACCC | 558 | TCCATAAAACATCCTCRC | 781 |
| CRAACCRTCTCCAACCCC | 559 | CCATAAAACATCCTCRCR | 782 |
| RAACCRTCTCCAACCCCR | 560 | CATAAAACATCCTCRCRT | 783 |
| AACCRTCTCCAACCCCRC | 561 | ATAAAACATCCTCRCRTT | 784 |
| ACCRTCTCCAACCCCRCR | 562 | TAAAACATCCTCRCRTTT | 785 |
| CCRTCTCCAACCCCRCRC | 563 | AAAACATCCTCRCRTTTC | 786 |
| CRTCTCCAACCCCRCRCC | 564 | AAACATCCTCRCRTTTCC | 787 |
| RTCTCCAACCCCRCRCCA | 565 | AACATCCTCRCRTTTCCC | 788 |
| TCTCCAACCCCRCRCCAA | 566 | ACATCCTCRCRTTTCCCR | 789 |
| CTCCAACCCCRCRCCAAC | 567 | CATCCTCRCRTTTCCCRA | 790 |
| TCCAACCCCRCRCCAACC | 568 | ATCCTCRCRTTTCCCRAA | 791 |
| CCAACCCCRCRCCAACCA | 569 | TCCTCRCRTTTCCCRAAA | 792 |
| CAACCCCRCRCCAACCAC | 570 | CCTCRCRTTTCCCRAAAA | 793 |
| AACCCCRCRCCAACCACC | 571 | CTCRCRTTTCCCRAAAAA | 794 |
| ACCCCRCRCCAACCACCC | 572 | TCRCRTTTCCCRAAAAAA | 795 |
| CCCCRCRCCAACCACCCR | 573 | CRCRTTTCCCRAAAAAAA | 796 |
| CCCRCRCCAACCACCCRA | 574 | RCRTTTCCCRAAAAAAAA | 797 |
| CCRCRCCAACCACCCRAA | 575 | CRTTTCCCRAAAAAAAAC | 798 |
| CRCRCCAACCACCCRAAA | 576 | RTTTCCCRAAAAAAAACR | 799 |
| RCRCCAACCACCCRAAAA | 577 | TTTCCCRAAAAAAAACRA | 800 |
| CRCCAACCACCCRAAAAA | 578 | TTCCCRAAAAAAAACRAA | 801 |
| RCCAACCACCCRAAAAAA | 579 | TCCCRAAAAAAAACRAAT | 802 |
| CCAACCACCCRAAAAAAC | 580 | CCCRAAAAAAAACRAATC | 803 |
| CAACCACCCRAAAAAACR | 581 | CCRAAAAAAAACRAATCC | 804 |
| AACCACCCRAAAAAACRT | 582 | CRAAAAAAAACRAATCCC | 805 |
| ACCACCCRAAAAAACRTT | 583 | RAAAAAAAACRAATCCCR | 806 |
| CCACCCRAAAAAACRTTC | 584 | AAAAAAAACRAATCCCRA | 807 |
| CACCCRAAAAAACRTTCT | 585 | AAAAAAACRAATCCCRAA | 808 |
| ACCCRAAAAAACRTTCTC | 586 | AAAAAACRAATCCCRAAA | 809 |
| CCCRAAAAAACRTTCTCA | 587 | AAAAACRAATCCCRAAAA | 810 |
| CCRAAAAAACRTTCTCAA | 588 | AAAACRAATCCCRAAAAA | 811 |
| CRAAAAAACRTTCTCAAC | 589 | AAACRAATCCCRAAAAAA | 812 |
| RAAAAAACRTTCTCAACC | 590 | AACRAATCCCRAAAAAAC | 813 |
| AAAAAACRTTCTCAACCT | 591 | ACRAATCCCRAAAAAACA | 814 |
| AAAAACRTTCTCAACCTC | 592 | CRAATCCCRAAAAAACAA | 815 |
| AAAACRTTCTCAACCTCC | 593 | RAATCCCRAAAAAACAAC | 816 |
| AAACRTTCTCAACCTCCA | 594 | AATCCCRAAAAAACAACC | 817 |
| AACRTTCTCAACCTCCAC | 595 | ATCCCRAAAAAACAACCT | 818 |
| ACRTTCTCAACCTCCACC | 596 | TCCCRAAAAAACAACCTA | 819 |
| CRTTCTCAACCTCCACCC | 597 | CCCRAAAAAACAACCTAA | 820 |
| RTTCTCAACCTCCACCCT | 598 | CCRAAAAAACAACCTAAT | 821 |
| TTCTCAACCTCCACCCTA | 599 | CRAAAAAACAACCTAATC | 822 |
| TCTCAACCTCCACCCTAA | 600 | RAAAAAACAACCTAATCT | 823 |
| CTCAACCTCCACCCTAAC | 601 | AAAAAACAACCTAATCTC | 824 |
| TCAACCTCCACCCTAACR | 602 | AAAAACAACCTAATCTCT | 825 |
| CAACCTCCACCCTAACRA | 603 | AAAACAACCTAATCTCTC | 826 |
| AACCTCCACCCTAACRAA | 604 | AAACAACCTAATCTCTCA | 827 |
| ACCTCCACCCTAACRAAC | 605 | AACAACCTAATCTCTCAA | 828 |
| CCTCCACCCTAACRAACC | 606 | ACAACCTAATCTCTCAAC | 829 |
| CTCCACCCTAACRAACCC | 607 | CAACCTAATCTCTCAACC | 830 |
| TCCACCCTAACRAACCCC | 608 | AACCTAATCTCTCAACCC | 831 |
| CCACCCTAACRAACCCCR | 609 | ACCTAATCTCTCAACCCT | 832 |
| CACCCTAACRAACCCCRC | 610 | CCTAATCTCTCAACCCTT | 833 |
| ACCCTAACRAACCCCRCR | 611 | CTAATCTCTCAACCCTTA | 834 |
| CCCTAACRAACCCCRCRC | 612 | TAATCTCTCAACCCTTAT | 835 |
| CCTAACRAACCCCRCRCC | 613 | AATCTCTCAACCCTTATA | 836 |
| CTAACRAACCCCRCRCCA | 614 | ATCTCTCAACCCTTATAA | 837 |
| TAACRAACCCCRCRCCAA | 615 | TCTCTCAACCCTTATAAA | 838 |
| AACRAACCCCRCRCCAAC | 616 | CTCTCAACCCTTATAAAA | 839 |
| ACRAACCCCRCRCCAACA | 617 | TCTCAACCCTTATAAAAA | 840 |
| CRAACCCCRCRCCAACAC | 618 | CTCAACCCTTATAAAAAA | 841 |
| RAACCCCRCRCCAACACT | 619 | TCAACCCTTATAAAAAAA | 842 |
| AACCCCRCRCCAACACTT | 620 | CAACCCTTATAAAAAAAA | 843 |
| ACCCCRCRCCAACACTTA | 621 | AACCCTTATAAAAAAAAA | 844 |
| CCCCRCRCCAACACTTAT | 622 | ACCCTTATAAAAAAAAAA | 845 |
| CCCRCRCCAACACTTATT | 623 | CCCTTATAAAAAAAAAAA | 846 |
| CCRCRCCAACACTTATTA | 624 | CCTTATAAAAAAAAAAAT | 847 |
| CRCRCCAACACTTATTAA | 625 | CTTATAAAAAAAAAAATA | 848 |
| RCRCCAACACTTATTAAC | 626 | TTATAAAAAAAAAAATAT | 849 |
| CRCCAACACTTATTAACC | 627 | TATAAAAAAAAAAATATC | 850 |
| RCCAACACTTATTAACCC | 628 | ATAAAAAAAAAAATATCA | 851 |
| CCAACACTTATTAACCCR | 629 | TAAAAAAAAAAATATCAA | 852 |
| CAACACTTATTAACCCRT | 630 | AAAAAAAAAAATATCAAA | 853 |
| AACACTTATTAACCCRTT | 631 | AAAAAAAAAATATCAAAA | 854 |
| ACACTTATTAACCCRTTA | 632 | AAAAAAAAATATCAAAAA | 855 |
| CACTTATTAACCCRTTAC | 633 | AAAAAAAATATCAAAAAC | 856 |
| ACTTATTAACCCRTTACC | 634 | AAAAAAATATCAAAAACA | 857 |
| CTTATTAACCCRTTACCA | 635 | AAAAAATATCAAAAACAA | 858 |
| TTATTAACCCRTTACCAC | 636 | AAAAATATCAAAAACAAA | 859 |
| TATTAACCCRTTACCACC | 637 | AAAATATCAAAAACAAAA | 860 |
| ATTAACCCRTTACCACCT | 638 | AAATATCAAAAACAAAAC | 861 |
| TTAACCCRTTACCACCTA | 639 | AATATCAAAAACAAAACR | 862 |
| TAACCCRTTACCACCTAT | 640 | ATATCAAAAACAAAACRA | 863 |
| AACCCRTTACCACCTATT | 641 | TATCAAAAACAAAACRAA | 864 |
| ACCCRTTACCACCTATTA | 642 | ATCAAAAACAAAACRAAA | 865 |
| CCCRTTACCACCTATTAA | 643 | TCAAAAACAAAACRAAAA | 866 |
| CCRTTACCACCTATTAAC | 644 | CAAAAACAAAACRAAAAC | 867 |
| CRTTACCACCTATTAACT | 645 | AAAAACAAAACRAAAACC | 868 |
| RTTACCACCTATTAACTT | 646 | AAAACAAAACRAAAACCA | 869 |
| TTACCACCTATTAACTTA | 647 | AAACAAAACRAAAACCAA | 870 |
| TACCACCTATTAACTTAA | 648 | AACAAAACRAAAACCAAA | 871 |
| ACCACCTATTAACTTAAC | 649 | ACAAAACRAAAACCAAAT | 872 |
| CCACCTATTAACTTAACC | 650 | CAAAACRAAAACCAAATC | 873 |
| CACCTATTAACTTAACCT | 651 | AAAACRAAAACCAAATCA | 874 |
| ACCTATTAACTTAACCTC | 652 | AAACRAAAACCAAATCAA | 875 |
| CCTATTAACTTAACCTCC | 653 | AACRAAAACCAAATCAAA | 876 |
| CTATTAACTTAACCTCCT | 654 | ACRAAAACCAAATCAAAT | 877 |
| TATTAACTTAACCTCCTT | 655 | CRAAAACCAAATCAAATC | 878 |
| ATTAACTTAACCTCCTTA | 656 | RAAAACCAAATCAAATCT | 879 |
| TTAACTTAACCTCCTTAC | 657 | AAAACCAAATCAAATCTC | 880 |
| TAACTTAACCTCCTTACA | 658 | AAACCAAATCAAATCTCT | 881 |
| AACTTAACCTCCTTACAC | 659 | AACCAAATCAAATCTCTT | 882 |
| ACTTAACCTCCTTACACT | 660 | ACCAAATCAAATCTCTTT | 883 |
| CTTAACCTCCTTACACTT | 661 | CCAAATCAAATCTCTTTC | 884 |
| TTAACCTCCTTACACTTC | 662 | CAAATCAAATCTCTTTCC | 885 |
| TAACCTCCTTACACTTCC | 663 | AAATCAAATCTCTTTCCT | 886 |
| AACCTCCTTACACTTCCA | 664 | AATCAAATCTCTTTCCTT | 887 |
| ACCTCCTTACACTTCCAT | 665 | ATCAAATCTCTTTCCTTA | 888 |
| CCTCCTTACACTTCCATT | 666 | TCAAATCTCTTTCCTTAA | 889 |
| CTCCTTACACTTCCATTT | 667 | CAAATCTCTTTCCTTAAA | 890 |
| TCCTTACACTTCCATTTC | 668 | AAATCTCTTTCCTTAAAC | 891 |
| CCTTACACTTCCATTTCA | 669 | AATCTCTTTCCTTAAACT | 892 |
| CTTACACTTCCATTTCAT | 670 | ATCTCTTTCCTTAAACTC | 893 |
| TTACACTTCCATTTCATT | 671 | TCTCTTTCCTTAAACTCC | 894 |
| TACACTTCCATTTCATTA | 672 | CTCTTTCCTTAAACTCCC | 895 |
| ACACTTCCATTTCATTAT | 673 | TCTTTCCTTAAACTCCCC | 896 |
| CACTTCCATTTCATTATT | 674 | CTTTCCTTAAACTCCCCA | 897 |
| | | TTTCCTTAAACTCCCCAA | 898 |

Within the nucleotide sequences set forth in table 1 "Y" represents C or T, wherein Y = C is specific for a methylated cytosine in the non-bisulfite converted DNA of the SNCA_{(-926/-483)} region of the human SNCA gene, and Y = T is specific for a non-methylated cytosine of a CpG dinucleotide in the non-bisulfite converted DNA of the SNCA_{(-926/-483)} region of the human SNCA gene.

Within the nucleotide sequences set forth in table 2 "R" represents G or A, wherein R = G is specific for a methylated cytosine in the non-bisulfite converted DNA of the SNCA_{(-926/-483)} region of the human SNCA gene, and R = A is specific for a non-methylated cytosine of a CpG dinucleotide in the non-bisulfite converted DNA of the SNCA_{(-926/-483)} region of the human SNCA gene.

For amplification reactions of the SNCA_{(-926/-483)} region of the human SNCA gene or at least of portions of SNCA_{(-926/-483)} region of the human SNCA gene comprising at least one of the 23 CpG dinucleotides present in the SNCA_{(-926/-483)} region of the human SNCA gene it is preferred that the forward oligonucleotide primer is selected from the group of oligonucleotides comprising or consisting of the nucleotide sequences selected from the group consisting of SEQ ID NO. 5 to SEQ ID NO: 451. For amplification reactions of the SNCA_{(-926/-483)} region of the human SNCA gene or at least of portions of SNCA_{(-926/-483)} region of the human SNCA gene comprising at least one of the 23 CpG dinucleotides present in the SNCA_{(-926/-483)} region of the human SNCA gene it is preferred that the reverse oligonucleotide primer is selected from the group of oligonucleotides comprising or consisting of the nucleotide sequences selected from the group consisting of SEQ ID NO. 452 to SEQ ID NO: 898.

Preferred primer for sequence analysis of the cloned PCR products from bisulfite converted DNA from the SNCA_{(-926/-483)} region of the human SNCA gene are selected from the group of oligonucleotides comprising or consisting of the nucleotide sequences selected from the group consisting of SEQ ID NO. 5 to SEQ ID NO: 451 and SEQ ID NO. 452 to SEQ ID NO: 898.

According to another preferred or additional embodiment of the first aspect of the invention, methylation of the human SNCA-gene, of intron 1 of the human SNCA gene, and/or of the SNCA_{(-926/-483)} region of the human SNCA gene, in particular of one or more CpG dinucleotides of the SNCA_{(-926/-483)} region of the human SNCA gene, preferably of one or more CpG dinculeotides of the SNCA_{(-926/-483)} region of the human SNCA gene selected from the group consisting of CpG dinucleotides No. 1, 2, 7, 8, 11, 12, 13, 18, 21, 22 and 23 of the SNCA_{(-926/-483)} region of the human SNCA gene, and more preferably of one or more CpG dinucleotides selected from the group consisting of CpG dinucleotides No. 1, 7, 8, 13, 22 and 23 of the SNCA_{(-926/-483)} region of the human SNCA gene, is determined by analyzing restriction enzyme digestions.

Analyzing restriction enzyme digestion of DNA to analyze the methylation status of said DNA is based on the property of some restriction enzymes to be unable to cut methylated DNA. Since in eukaryotic DNA only cytosine in CG context can be methylated, restriction enzymes with CG sequences within their restriction sites come may be used. Two classical enzyme pairs are for example HpaII - MspI (CCGG) and SmaI - XmaI (CCCGGG). Both enzymes of each pair recognize nucleotides sequences comprising a CCGG sequence. However HpaII and SmaI are unable to cut DNA when the internal cytosine is methylated. This property makes HpaII - MspI and SmaI - XmaI valuable tools for rapid methylation analysis.

Prior to the methylation analysis by restriction enzyme digestion, the nucleotide sequence of the DNA fragment to be investigated has to be analyzed for the presence of CpG dinucleotides and specific restriction sites for enzyme pairs wherein one isoschizomer hydrolyses the DNA even if the cytosine of the CpG dinucleotide within the recognition sequence is methylated, and wherein the other isoschizomer does not. After restriction enzyme digestion, the DNA fragments will be separated by gel electrophoresis. A Southern blot hybridization of the separated DNA fragments has to be performed, wherein the probe for used for Southern blot hybridization should be located within the region to be analyzed and cover it completely or at least partially.

According to the second aspect of the present invention, a method of determining the methylation pattern of the human SNCA gene for diagnosing and/or assessing a patient's risk of developing Parkinson's disease is provided, in particular of developing sporadic PD, wherein the SNCA gene is obtained from DNA of peripheral blood monocytes of said patient. The method according to the second aspect permits to establish a method for diagnosing Parkinson's disease, even if no neurological symptoms associated with Parkinson's disease are present in the patient yet. The method according to the second aspect of the present invention as well as the diagnostic assay that is based on the method according to the second aspect of the present invention are inexpensive and easy to perform without affecting the patient's acceptance.

In a preferred embodiment of the method according to the second aspect of the present invention, the methylation pattern of intron 1 of the human SNCA gene from genomic DNA of the patient's peripheral blood monocytes is determined.

In another preferred or additional embodiment of the method according to the second aspect of the present invention, the methylation pattern of the SNCA_{(-926/-483)} region of the human SNCA gene from genomic DNA of the patient's peripheral blood monocytes is determined.

In another preferred or additional embodiment of the method according to the second aspect of the present invention, the methylation status of one or more CpG dinucleotides of the SNCA_{(-926/-483)} region of the human SNCA gene is determined. Preferably said one or more CpG dinucleotides is/are selected from the group consisting of CpG dinucleotides No. 1, 2, 7, 8, 11, 12, 13, 18, 21, 22 and 23 of the SNCA_{(-926/-483)} region of the human SNCA gene. More preferably said one or more CpG dinucleotides is/are selected from the group consisting of CpG dinucleotides No. 1, 7, 8, 13, 22 and 23 of the SNCA_{(-926/-483)} region of the human SNCA gene.

Notwithstanding that virtually any method for analyzing the methylation status of a given CpG dinucleotide can be employed for analyzing the methylation pattern of the human SNCA gene, of intron 1 of the human SNCA gene, and/or of the SNCA_{(-926/-483)} region of the human SNCA gene, in particular of one or more CpG dinucleotides of the SNCA_{(-926/-483)} region of the human SNCA gene, preferably of one or more CpG dinculeotides of the SNCA_{(-926/-483)} region of the human SNCA gene selected from the group consisting of CpG dinucleotides 1, 2, 7, 8, 11, 12, 13, 18, 21, 22 and 23 of the SNCA_{(-926/-483)} region of the human SNCA gene, and more preferably said one or more CpG dinucleotides is/are selected from the group consisting of CpG dinucleotides No. 1, 7, 8, 13, 22 and 23 of the SNCA_{(-926/-483)} region of the human SNCA gene, it is preferred that the methylation status is determined by one or more methods selected from the group consisting of methylation specific PCR, direct sequence analysis of bisulfite-treated DNA, COBRA-Assay and methylation-specific restriction enzyme digestion as described in more detail herein above.

In a preferred embodiment of the method according to the second aspect of the invention, methylation of the human SNCA-gene, of intron 1 of the human SNCA gene, and/or of the SNCA_{(-926/-483)} region of the human SNCA gene, in particular of one or more CpG dinucleotides of the SNCA_{(-926/-483)} region of the human SNCA gene is determined by methylation specific PCR (MSP). Preferably said one or more CpG dinucleotides of the SNCA_{(-926/-483)} region of the human SNCA gene is/are selected from the group consisting of CpG dinucleotides 1, 2, 7, 8, 11, 12, 13, 18, 21, 22 and 23 of the SNCA_{(-926/-483)} region of the human SNCA gene, and more preferably said one or more CpG dinucleotides is/are selected from the group consisting of CpG dinucleotides No. 1, 7, 8, 13, 22 and 23 of the SNCA_{(-926/-483)} region of the human SNCA gene,

In another preferred or additional embodiment of the method according to the second aspect of the invention, methylation of the human SNCA-gene, of intron 1 of the human SNCA gene, and/or of the SNCA_{(-926/-483)} region of the human SNCA gene, in particular of one or more CpG dinucleotides of the SNCA_{(-926/-483)} region of the human SNCA gene is determined by sequence analysis of bisulfite treated DNA. Preferably said one or more CpG dinucleotides of the SNCA_{(-926/-483)} region of the human SNCA gene is/are selected from the group consisting of CpG dinucleotides 1, 2, 7, 8, 11, 12, 13, 18, 21, 22 and 23 of the SNCA_{(-926/-483)} region of the human SNCA gene, and more preferably said one or more CpG dinucleotides is/are selected from the group consisting of CpG dinucleotides No. 1, 7, 8, 13, 22 and 23 of the SNCA_{(-926/-483)} region of the human SNCA gene,

In another preferred or additional embodiment of the method according to the second aspect of the invention, methylation of the human SNCA-gene, of intron 1 of the human SNCA gene, and/or of the SNCA_{(-926/-483)} region of the human SNCA gene, in particular of one or more CpG dinucleotides of the SNCA_{(-926/-483)} region of the human SNCA gene is determined by combined bisulfite restriction analysis (COBRA assay). Preferably said one or more CpG dinucleotides of the SNCA_{(-926/-483)} region of the human SNCA gene is/are selected from the group consisting of CpG dinucleotides 1, 2, 7, 8, 11, 12, 13, 18, 21, 22 and 23 of the SNCA_{(-926/-483)} region of the human SNCA gene, and more preferably said one or more CpG dinucleotides is/are selected from the group consisting of CpG dinucleotides No. 1, 7, 8, 13, 22 and 23 of the SNCA_{(-926/-483)} region of the human SNCA gene,.

In another preferred or additional embodiment of the method according to the second aspect of the invention, methylation of the human SNCA-gene, of intron 1 of the human SNCA gene, and/or of the SNCA_{(-926/-483)} region of the human SNCA gene, in particular of one or more CpG dinucleotides of the SNCA_{(-926/-483)} region of the human SNCA gene is determined by analyzing restriction enzyme digestions. Preferably said one or more CpG dinucleotides of the SNCA_{(-926/-483)} region of the human SNCA gene is/are selected from the group consisting of CpG dinucleotides 1, 2, 7, 8, 11, 12, 13, 18, 21, 22 and 23 of the SNCA_{(-926/-483)} region of the human SNCA gene, and more preferably said one or more CpG dinucleotides is/are selected from the group consisting of CpG dinucleotides No. 1, 7, 8, 13, 22 and 23 of the SNCA_{(-926/-483)} region of the human SNCA gene,.

In a third aspect, the present invention further comprises a method for diagnosis and/or assessment of a patient's risk of developing Parkinson's disease, in particular sporadic PD, wherein said diagnostic method comprises analysis of the methylation pattern of the human SNCA gene from DNA of peripheral blood monocytes, said peripheral blood monocytes being obtained from said patient.

The method for diagnosing Parkinson's disease and/or assessing a patient's risk of developing Parkinson's disease according to the third aspect of the present invention may comprise obtaining a blood sample from said patient. Said blood sample may be specifically obtained for diagnosing Parkinson's disease or assessing the patient's risk of developing Parkinson's disease. Alternatively, blood samples of said patient may be used which were obtained from said patient for other reasons and/or earlier, wherein said blood sample had to be stored under appropriate conditions which prevent the genomic DNA from the peripheral blood monocytes of said blood sample from becoming degraded.

The method according to the third aspect of the present invention comprises isolating of peripheral blood monocytes from the patient's blood sample or blood samples. The method according to the third aspect of the present invention further comprises isolating genomic DNA from the patient's peripheral blood monocytes, and determining the methylation pattern of the patient's SNCA gene, preferably the methylation pattern of intron 1 of the patient's SNCA gene, more preferably the methylation pattern of the SNCA_{(-926/-483)} region of the patient's SNCA gene. In a particularly preferred embodiment of the method according to the third aspect of the present invention, the methylation status of one or more CpG dinucleotides of the SNCA_{(-926/-483)} region of the patient's SNCA gene is determined. Preferably the methylation status of one or more CpG dinucleotides is determined, wherein said one or more CpG dinucleotides is/are selected from the group consisting of CpG dinucleotides No. 1, 2, 7, 8, 11, 12, 13, 18, 21, 22 and 23 of the SNCA_{(-926/-483)} region of the patient's SNCA gene is determined, and more preferably said one or more CpG dinucleotides is/are selected from the group consisting of CpG dinucleotides No. 1, 7, 8, 13, 22 and 23 of the SNCA_{(-926/-483)} region of the human SNCA gene.

Determining the methylation pattern of the patient's SNCA gene, of intron 1 of the patient's SNCA gene, and/or of the SNCA_{(-926/-483)} region of the patient's SNCA gene, more specifically the methylation status of one or more CpG dinucleotides of the SNCA_{(-926/-483)} region of the patient's SNCA gene, preferably of one or more CpG dinucleotides of the SNCA_{(-926/-483)} region of the patient's SNCA gene selected from the group consisting of CpG dinucleotides No. 1, 2, 7, 8, 11, 12, 13, 18, 21, 22 and 23 of the SNCA_{(-926/-483)} region of the patient's SNCA gene, and more preferably of one or more CpG dinucleotides selected from the group consisting of CpG dinucleotides No. 1, 7, 8, 13, 22 and 23 of the SNCA_{(-926/-483)} region of the patient's SNCA gene, may utilize virtually any technique that is suitable for analyzing a DNA's methylation status. However, according to a preferred embodiment of the method according to the third aspect of the present invention, the methylation status is determined by one or more methods selected from the group consisting of methylation specific PCR, sequence analysis of bisulfite-treated DNA, COBRA-Assay and methylation-specific restriction patterns.

In a preferred embodiment, the method according to the third aspect of the present invention further comprises comparing the methylation pattern of the patient's SNCA gene, of intron 1 of the patient's SNCA gene, and/or of the SNCA_{(-926/-483)} region of the patient's SNCA gene, more specifically the methylation status of one or more CpG dinucleotides of the SNCA_{(-926/-483)} region of the patient's SNCA gene, preferably of one or more CpG dinucleotides of the SNCA_{(-926/-483)} region of the patient's SNCA gene selected from the group consisting of CpG dinucleotides No. 1, 2, 7, 8, 11, 12, 13, 18, 21, 22 and 23 of the SNCA_{(-926/-483)} region of the patient's SNCA gene, and more preferably of one or more CpG dinucleotides selected from the group consisting of CpG dinucleotides No. 1, 7, 8, 13, 22 and 23 of the SNCA_{(-926/-483)} region of the patient's SNCA gene, with the methylation status of the corresponding SNCA gene, intron, region and/or CpG dinucleotide(s) of healthy individuals.

In yet another preferred embodiment, the method according to the third aspect of the present invention further comprises comparing the methylation pattern of the patient's SNCA gene, of intron 1 of the patient's SNCA gene, and/or of the SNCA_{(-926/-483)} region of the patient's SNCA gene, more specifically of one or more CpG dinucleotides of the SNCA_{(-926/-483)} region of the patient's SNCA gene, preferably the methylation status of one or more CpG dinucleotides of the SNCA_{(-926/-483)} region of the patient's SNCA gene selected from the group consisting of CpG dinucleotides No. 1, 2, 7, 8, 11, 12, 13, 18, 21, 22 and 23 of the SNCA_{(-926/-483)} region of the patient's SNCA gene, and more preferably of one or more CpG dinucleotides selected from the group consisting of CpG dinucleotides No. 1, 7, 8, 13, 22 and 23 of the SNCA_{(-926/-483)} region of the patient's SNCA gene, from DNA of a blood sample obtained from the patient at a given point of time with the methylation status of the corresponding gene, intron, region and/or CpG dinucleotide(s) from DNA of one or more further blood samples of the same patient, wherein said one or more further blood sample(s) was/were obtained at different points of time than said blood sample obtained from the patient at said given point of time. This embodiment permits to monitor alterations in the methylation pattern of the patient's SNCA gene, and/or of the SNCA_{(-926/-483)} region of the patient's SNCA gene, more specifically of one or more CpG dinucleotides of the SNCA_{(-926/-483)} region of the patient's SNCA gene, preferably of one or more CpG dinucleotides of the SNCA_{(-926/-483)} region of the patient's SNCA gene selected from the group consisting of CpG dinucleotides No. 1, 2, 7, 8, 11, 12, 13, 18, 21, 22 and 23 of the SNCA_{(-926/-483)} region of the patient's SNCA gene, and more preferably of one or more CpG dinucleotides selected from the group consisting of CpG dinucleotides No. 1, 7, 8, 13, 22 and 23 of the SNCA_{(-926/-483)} region of the patient's SNCA gene, over time, and may enable to elucidating the etiology of Parkinson's disease and/or monitoring progression or regression of Parkinson's disease of a patient.

The method according to the third aspect of the present invention further comprises determining whether the patient's SNCA gene, intron 1 of the patient's SNCA gene, or the SNCA_{(-926/-483)} region of the patient's SNCA gene is hypomethylated compared to the corresponding gene, intron or region of the SNCA gene from healthy individuals. Hypomethylation of the patient's SNCA gene, of intron 1 of the patient's SNCA gene, or of the SNCA_{(-926/-483)} region of the patient's SNCA gene may be determined in that the methylation status of one or more CpG dinucleotides of the SNCA_{(-926/-483)} region of the patient's SNCA gene is compared to the methylation status of the corresponding CpG dinucleotide(s) of the SNCA_{(-926/-483)} region from healthy individuals. In a preferred embodiment, hypomethylation is determined in that the methylation status of one or more CpG dinucleotides of the SNCA_{(-926/-483)} region of the patient's SNCA gene selected from the group consisting of CpG dinucleotides No. 1, 7, 8, 13, 22 and 23 of the SNCA_{(-926/-483)} region of the patient's SNCA gene is compared to the methylation status of the corresponding CpG dinucleotide(s) of the SNCA_{(-926/-483)} region from healthy individuals.

In determining whether the patient's SNCA gene, intron 1 of the patient's SNCA gene or the SNCA_{(-926/-483)} region of the patient's SNCA gene is hypomethylated compared to the corresponding gene, intron or region of healthy individuals it can be deduced whether the patient is at risk of developing Parkinson's disease even if neurological symptoms are absent, or whether the patient already has Parkinson's disease even no neurological symptoms are present or only present mildly and yet non-specifically.

In another or additional embodiment of the third aspect of the present invention, the method comprises determining whether the patient's SNCA gene, intron 1 of the patient's SNCA gene, or the SNCA_{(-926/-483)} region of the patient's SNCA gene is hypermethylated with respect to particular CpG dinucleotides within said SNCA gene, said intron 1 or said SNCA_{(-926/-483)} region compared to the corresponding gene, intron or region of the SNCA gene from healthy individuals. Hypermethylation of said SNCA gene, said intron 1 or said SNCA_{(-926/-483)} region with respect to particular CpG dinucleotides therein may be determined in that the methylation status of one or more CpG dinucleotides of the SNCA_{(-926/-483)} region of the patient's SNCA gene is compared to the methylation status of the corresponding CpG dinucleotide(s) of the SNCA_{(-926/-483)} region from healthy individuals. In a preferred embodiment, hypermethylation is determined in that the methylation status of one or more CpG dinucleotides selected from the group consisting of CpG dinucleotides No. 2, 11, 12, 18 and 21 of the SNCA_{(-926/-483)} region of the patient's SNCA gene is/are compared to the methylation status of the corresponding CpG dinucleotide(s) of the SNCA_{(-926/-483)} region from healthy individuals.

In determining whether the patient's SNCA gene, intron 1 of the patient's SNCA gene or the SNCA_{(-926/-483)} region of the patient's SNCA gene is hypermethylated with respect to particular CpG dinucleotides within the SNCA gene, intron 1 of the SNCA gene or the SNCA_{(-926/-483)} region compared to the corresponding gene, intron or region of healthy individuals it can be deduced whether the patient is at risk of developing Parkinson's disease even if neurological symptoms are absent, or whether the patient already has Parkinson's disease even no neurological symptoms are present or only present mildly and yet non-specifically.

The present invention will now be described with respect to particular embodiments and with reference to the figures, but the invention is not limited thereto, but only to the claims. The drawings described are only schematic and are non-limiting.

Figure 1 displays the nucleotide sequence of SNCA_{(-926/-483)} (SEQ ID No. 1) which is a fragment of intron I of the human SNCA gene, ranging from nucleotide position -926 to nucleotide position -483 upstream of the SNCA start codon (nucleotide positions 1 to 3). Within SNCA_{(-926/-483)} 23 CpG dinucleotides are present which may be subject to methylation. Said 23 CpG dinucleotides are identified in the nucleotide sequence in that they are displayed in bold letters. The 23 CpG dinucleotides are consecutively enumerated, from distant to proximal with respect to the SNCA start codon. Predicted transcription factor binding sites within the SNCA_{(-926/-483)} adjacent to CpG dinucleotides are designated below the nucleotide sequence.

Figure 2 shows a diagram illustrating the methylation pattern of the 23 CpG dinucleotides within the SNCA_{(-926/-483)} region from peripheral blood monocytes DNA. White columns display the percentage of methylation of the cytosine of the CpG dinucleotide indicated on the X-axis from healthy individuals whereas black columns display the percentage of methylation of the cytosine of the CpG dinucleotide indicated on the X-axis from patients affected with sporadic PD. Mean values are indicated by the height of each column and standard deviations are indicated. Asterisks indicate statistical significance of the obtained results with **p*<0.05 and ***p*<0.01.

### Examples

Peripheral blood monocytes were isolated from blood samples of 19 patients suffering from sporadic PD and 20 healthy individuals. DNA isolation and bisulfite treatment of the isolated DNA were performed as described by Pieper et al. (Pieper, H.C. et al. (2008) Different methylation of the TNF-alpha promoter in cortex and substantia nigra: implications for selective neuronal vulnerability. Neurobiol. Dis. 32:521-527).

Bisulfite treated DNA was amplified using SNCA-For (SEQ ID NO: 2) and SNCA-Rev (SEQ ID NO: 3), specifically designed for bisulfite treated SNCA_{(-926/-483)} DNA. PCR products were cloned into pCR 2.1 TOPO vector (Invitrogen) following the manufacturer's instructions. Plasmid DNA was isolated from at least ten clones per individual, and sequenced using vector specific primer M13reverse (CAGGAAACAGCTATGAC; SEQ ID NO: 4) and the Big Dye Terminator v1.1 Cycle Sequencing Kit (Applied Biosystems) and analyzed on an ABI PRISM 310 Genetic Analyzer.

The results of determining the methylation pattern of the SNCA_{(-926/-483)} DNA from lymphocytes of healthy individuals and PD patients is shown in Figure 2.

Figure 2 compares the methylation status of the 23 CpG dinucleotides within the SNCA_{(-926/-483)} region of the human SNCA gene from peripheral blood monocytes of patients who developed Parkinson's disease with those of healthy individuals. As can be inferred from Figure 2, the percentage of methylation of CpG dinucleotide Nos. 1, 7, 8, 13, 22 and 23 is significantly lower in patients affected with PD compared to healthy control individuals. Hence, the SNCA gene, in particular intron 1 of the human SNCA genes, more specifically the SNCA_{(-926/-483)} region, is significantly hypomethylated with respect to CpG dinucleotides No. 1, 7, 8, 13 and 23. Thus, non-methylation of one or more dinucleotides selected from the group consisting of CpG dinucleotides No. 1, 7, 8, 13, 22 and 23 of the SNCA_{(-926/-483)} region obtained from DNA of peripheral blood monocytes of a patient may indicate that said patient is at higher risk of developing Parkinson's disease or already developed Parkinson's disease even if no symptoms are present at that stage of the disease.

In addition, Figure 2 indicates that the percentage of CpG dinucleotides No. 2, 11, 12, 18 and 21 being methylated is higher in the SNCA_{(-926/-483)} region of the human SNCA gene obtained from peripheral blood monocytes of PC patients than in that obtained from healthy individuals. Although statistically not significant yet, the tendency is striking, and analyzing additional samples will render this observation statistically significant. Thus, methylation of CpG dinucleotides No. 2, 11, 12, 18 and 21 of the SNCA_{(-926/-483)} region obtained from DNA of peripheral blood monocytes of a patient may indicate that said patient is at higher risk of developing Parkinson's disease or already developed Parkinson's disease even if no symptoms are present at that stage of the disease.

Overall, methylation of the human SNCA gene from peripheral blood monocytes, in particular of intron 1 of the human SNCA genes, and more specifically of the SNCA_{(-926/-483)} region, is reduced in PD patients compared to control. Furthermore, linear regression analysis denoted a trend towards hypomethylation with increased age in PD patients.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. Use of peripheral blood monocytes of a patient for diagnosing and/or assessing the patient's risk of developing Parkinson's disease, **characterized in that** the methylation pattern of the SNCA gene of the DNA of said peripheral blood monocytes is determined.

2. Use according to claim 1, **characterized in that** the methylation pattern of Intron I of the SNCA gene is determined.

3. Use according to claim 1 or 2, **characterized in that** the methylation pattern of the SNCA_{(-926/-483)} region is determined.

4. Use according to any one of claims 1 to 3, **characterized in that** the methylation status of one or more CpG dinucleotides of the SNCA_{(-926/-483)} region is determined, wherein said CpG dinucleotide(s) is/are selected from the group consisting of CpG dinucleotides No. 1, 2, 7, 8, 11, 12, 13, 18, 21, 22 and 23.

5. Use according to any one of claims 1 to 4, **characterized in that** the methylation status is determined by one or more methods selected from the group consisting of methylation specific PCR, sequence analysis of bisulfite-treated DNA, COBRA-Assay and methylation-specific restriction patterns.

6. Method of determining the methylation pattern of the human SNCA gene for diagnosing and/or assessing a patient's risk of developing Parkinson's disease, **characterized in that** the SNCA gene is obtained from DNA of peripheral blood monocytes of said patient.

7. Method according to claim 6, **characterized in that** the methylation pattern of intron I of the SNCA gene is determined.

8. Method according to claim 6 or 7, **characterized in that** the methylation pattern of the SNCA_{(-926/-483)} region of the SNCA gene is determined.

9. Method according to any one of claims 6 to 8, **characterized in that** the methylation status of one or more CpG dinucleotides of the SNCA_{(-926/-483)} region is determined, wherein said CpG dinucleotide(s) is/are selected from the group consisting of CpG dinucleotides No. 1, 2, 7, 8, 11, 12, 13, 18, 21, 22 and 23.

10. Method according to any one of claims 6 to 9, **characterized in that** that the methylation status is determined by one or more methods selected from the group consisting of methylation specific PCR, sequence analysis of bisulfite-treated DNA, COBRA-Assay and methylation-specific restriction patterns.
